(19) Europäisches Patentamt
European Patent Office
Office européen des brevets

(11) EP 2 185 520 B1

(12) **EUROPEAN PATENT SPECIFICATION**

(45) Date of publication and mention of the grant of the patent:
**26.12.2012 Bulletin 2012/52**

(21) Application number: **08801822.1**

(22) Date of filing: **03.09.2008**

(51) Int Cl.:
***C07D 215/20*** *(2006.01)* ***A01N 43/42*** *(2006.01)*

(86) International application number:
**PCT/EP2008/007195**

(87) International publication number:
**WO 2009/030469 (12.03.2009 Gazette 2009/11)**

(54) **FUNGICIDAL 2-ALKYLTHIO-2-QUINOLINYLOXY-ACETAMIDE DERIVATIVES**

2-ALKYLTHIO-2-CHINOLINYLOXY-ACETAMIDDERIVATE ALS FUNGIZIDE

DÉRIVÉS FONGICIDES DE 2-ALKYLTHIO-2-QUINOLÉINYLOXY-ACÉTAMIDE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **05.09.2007 GB 0717258**

(43) Date of publication of application:
**19.05.2010 Bulletin 2010/20**

(73) Proprietor: **Syngenta Participations AG**
**4058 Basel (CH)**

(72) Inventors:
- **BEAUDEGNIES, Renaud**
  **CH-4332 Stein (CH)**
- **MURPHY, Kessabi, Fiona**
  **CH-4332 Stein (CH)**
- **QUARANTA, Laura**
  **CH-4332 Stein (CH)**
- **BRUNNER, Hans-Georg**
  **CH-4415 Lausen (CH)**
- **CEDERBAUM, Fredrik**
  **CH-4332 Stein (CH)**

(74) Representative: **Hölscher, Ingo**
**Syngenta Crop Protection Münchwilen AG**
**Intellectual Property**
**Werk Stein**
**Schaffhauserstrasse**
**4332 Stein (CH)**

(56) References cited:
**WO-A-2004/108663**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

This invention relates to novel *N*-alkynyl-2-alkylthio-2-(substituted heteroaryloxy)-alkylamides and to their sulphinyl and sulphonyl derivatives. It also relates to processes for preparing them, to compositions containing them and to methods of using them to combat fungi, especially fungal infections of plants.

Certain *N*-alkynyl-2-alkylthio-2-(substituted heteroaryloxy)alkylamides and their sulphinyl and sulphonyl derivatives are described, for example, in WO 04/108663 as being useful as fungicides.

The present invention is concerned with the provision of new *N*-alkynyl-2-alkylthio-2-(3-halo-8-alkyl-substituted heteroaryloxy)alkylamides and their sulphinyl and sulphonyl derivatives with improved properties as plant fungicides.

Thus, according to the present invention there is provided a compound of the general formula (1)

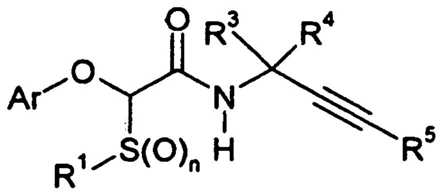

(1) ,

wherein Ar is a group of the formula (A):

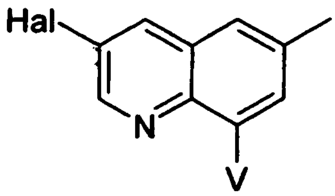

(A) ,

wherein

Hal is chloro, bromo or iodo
V is methyl or ethyl, where V is different from methyl when Hal is iodo,
$R^1$ is methyl or ethyl;
$R^3$ and $R^4$ are independently H, $C_{1-3}$ alkyl, $C_{2-3}$ alkenyl or $C_{2-3}$ alkynyl, or
$R^3$ and $R^4$ join with the carbon atom to which they are attached to form a 4 to 5-membered carbocyclic ring optionally containing one O, S or N atom and optionally substituted with halo or $C_{1-4}$ alkyl;
$R^5$ is H, $C_{1-4}$ alkyl or $C_{3-6}$ cycloalkyl or $C_{3-6}$ cycloalkoxy or $C_{2-4}$ alkenyl, in which the alkyl or cycloalkyl or cycloalkoxy or alkenyl group is optionally substituted with halo, hydroxy, $C_{1-6}$ alkyl, $C_{1-6}$ alkoxy, $C_{1-4}$ alkoxy-$C_{1-4}$ alkoxy, cyano, $C_{3-5}$ alkenyloxy or $C_{3-5}$ alkynyloxy, and n is 0, 1 or 2.

For the avoidance of doubt, the unattached single bond shown in the group of formula (A) indicates the point of attachment of the Ar group in the compound of formula (1) to the rest of the molecule.

The compounds of the invention contain at least one asymmetric carbon atom (and at least two when $R^3$ and $R^4$ are different) and may exist as enantiomers (or as pairs of diastereoisomers) or as mixtures of such. Further, when n is 1, the compounds of the invention are sulphoxides, which can exists in two enantiomeric forms, and the adjacent carbon can also exists in two enantiomeric forms. Compounds of general formula (1) can therefore exist as racemates, diastereoisomers, or single enantiomers, and the invention includes all possible isomers or isomer mixtures in all proportions. It is to be expected that for any given compound, one isomer may be more fungicidally active than another.

In a preferred group of compounds of the formula (1) $R^3$ and $R^4$ are independently H, $C_{1-3}$ alkyl, $C_{2-3}$ alkenyl or $C_{2-3}$ alkynyl provided that when both are other than H their combined total of carbon atoms does not exceed 4.

Preferably, in the compounds of the formula (1) Hal is chloro or bromo.

Further, it is preferred when V is methyl.

Preferably, $R^3$ and $R^4$ are independently H or $C_{1-3}$ alkyl or $R^3$ and $R^4$ join with the carbon atom to which they

are attached to form a 4 or 5-membered carbocyclic ring.

More preferably, $R^3$ and $R^4$ are independently H or $C_{1-3}$ alkyl, and in particular, $R^3$ and $R^4$ are both methyl.

Preferably, $R^3$ and $R^4$ join with the carbon atom to which they are attached to form a 4 or 5-membered carbocyclic ring.

In a preferred group of compounds of the formula (1) $R^5$ is H, $C_{1-4}$ alkyl, $C_{3-6}$ cycloalkyl, $C_{2-6}$ alkoxyalkyl, $C_{3-6}$ alkenyloxyalkyl, $C_{3-6}$ alkynyloxyalkyl, $C_{1-4}$ hydroxyalkyl. or $C_{1-4}$ haloalkyl.

In another preferred group of compounds of the formula (1) Hal is bromo, V is methyl, R' is methyl, n is 0, $R^3$ and $R^4$ are methyl and $R^5$ is H, $C_{1-4}$ alkyl, $C_{3-6}$ cycloalkyl, $C_{1-6}$ alkoxy-$C_{1-4}$ alkyl, $C_{3-6}$ alkenyloxy-$C_{1-4}$ alkyl, $C_{3-6}$ alkynyloxy-$C_{1-4}$ alkyl, $C_{1-4}$ hydroxyalkyl, or $C_{1-4}$ haloalkyl.

It is preferred when $R^5$ is H.

It is also preferred when $R^5$ is $C_{1-4}$ alkyl.

It is also preferred when $R^5$ is methyl.

It is also preferred when $R^5$ is ethyl.

It is also preferred when $R^5$ is $C_{1-3}$ alkoxy-$C_{1-3}$ alkyl.

It is also preferred when $R^5$ is $C_{3-4}$ alkenyloxy-$C_{1-3}$ alkyl.

It is also preferred when $R^5$ is $C_{3-4}$ alkynyloxy-$C_{1-3}$ alkyl.

It is also preferred when $R^5$ is $C_{1-3}$ hydroxyalkyl.

It is also preferred when $R^5$ is $C_{1-3}$ haloalkyl.

It is also preferred when $R^5$ is $CH_2OH$, $CH_2OMe$, $CH_2OEt$, $CH_2OCH_2CHCH_2$, $CH_2OCH_2CCH$, $(CH_2)_2OMe$.

Compounds that form part of the invention are illustrated in Tables 1 and 2 below. Characterising data is given in Table 4 after the Examples.

Table 1

| The compounds in Table 1 are of the general formula (1) where Ar is a group of the formula (A), Hal is Cl, V is methyl, R' is methyl, $R^3$ and $R^4$ are both methyl, n is 0 and $R_5$ has the values given in the Table. | |
|---|---|
| Compound No. | $R_5$ |
| 1 | H |
| 2 | $CH_3$ |
| 3 | $C_2H_5$ |
| 4 | *n*-$C_3H_7$ |
| 5 | *i*-$C_3H_7$ |
| 6 | *n*-$C_4H_9$ |
| 7 | *sec*-$C_4H_9$ |
| 8 | *iso*-$C_4H_9$ |
| 9 | *tert*-$C_4H_9$ |
| 10 | cyclopropyl |
| 11 | cyclobutyl |
| 12 | cyclopentyl |
| 13 | $FCH_2$ |
| 14 | $F_2CH$ |
| 15 | $FCH_2CH_2$ |
| 16 | $F_2CHCH_2$ |
| 17 | $ClCH_2$ |
| 18 | $Cl_2CH$ |
| 19 | $ClCH_2CH_2$ |
| 20 | $Cl_2CHCH_2$ |

(continued)

| Compound No. | $R_5$ |
|---|---|
| 21 | $HOCH_2$ |
| 22 | $HOCH_2CH_2$ |
| 23 | $CH_3OCH_2$ |
| 24 | $CH_3OCH_2CH_2$ |
| 25 | $C_2H_5OCH_2$ |
| 26 | $C_2H_5OCH_2CH_2$ |
| 27 | $CH_3(CH_3O)CH$ |
| 28 | *n*-$C_3H_7OCH_2$ |
| 29 | *n*-$C_3H_7OCH_2CH_2$ |
| 30 | *t*-$C_4H_9OCH_2$ |
| 31 | *t*-$C_4H_9OCH_2CH_2$ |
| 32 | NC-$C_2H_4$ |
| 33 | NC-*n*-$C_3H_8$ |
| 34 | NC-*n*-$C_4H_8$ |
| 35 | $(CH_3)_2C(CN)CH_2$ |
| 36 | allyl$OCH_2$ |
| 37 | allyl$OCH_2CH_2$ |
| 38 | allyl$OCH_2CH_2CH_2$ |
| 39 | propargyl$OCH_2$ |
| 40 | propargyl$OCH_2CH_2$ |
| 41 | propargyl$OCH_2CH_2CH_2$ |
| 42 | $CH_3OCH_2CH_2OCH_2$ |
| 43 | $CH_3OCH_2CH_2OCH_2CH_2$ |
| 44 | $C_2H_5OCH_2CH_2OCH_2$ |
| 45 | $C_2H_5OCH_2CH_2OCH_2CH_2$ |
| 46 | $CH_3OCH_2OCH_2$ |
| 47 | $C_2H_5OCH_2OCH_2$ |
| 48 | *tert*-$C_4H_9(CH_3)_2SiOCH_2$ |
| 49 | *tert*-$C_4H_9(CH_3)_2SiOC_2H_4$ |
| 50 | Cl-*n*-$C_3H_6$ |
| 51 | F-*n*-$C_3H_6$ |
| 52 | $BrCH_2$ |
| 53 | $BrCH_2CH_2$ |
| 54 | Br-*n*-$C_3H_6$ |
| 55 | $CH_3OCH_2CH_2OCH_2OCH_2$ |
| 56 | Tetrahydropyran-2-yl$OCH_2$ |
| 57 | Tetrahydrofuran-2-yl$OCH_2$ |
| 58 | Tetrahydrofuran-2-yl$CH_2$ |

(continued)

| Compound No. | $R_5$ |
|---|---|
| 59 | Oxiran-2-yl |
| 60 | Oxetan-2-yl |

Table 2

Table 2 consists of 60 compounds of the general formula (1) where Ar is a group of the formula (A), Hal is Br, V is methyl, $R^1$ is methyl, $R^3$ and $R^4$ are both methyl, n is 0, and $R_5$ has the values given in the Table 1.

Table 4

| This table gives characterising data (physical/spectral data) for compounds that have been prepared and are in part listed in Tables 1-2. | | | | | | | |
|---|---|---|---|---|---|---|---|
| Compound No | Hal | V | $R^1$ | $R^3$ | $R^4$ | $R^5$ | Physical/ spectral data |
| 1 | Br | Me | Me | Me | Me | Me | m.p. = 122-123°C |
| 2 | Br | Me | Me | Me | Me | H | m.p. = 136-137°C |
| 3 | Br | Me | Me | Me | Me | $CH_2OMe$ | m.p. = 128-130°C |
| 4 | Br | Me | Me | Me | Me | Et | m.p. = 87-90°C |
| 5 | Br | Me | Me | Me | Me | $CH_2CH_2OMe$ | m.p. = 128-130°C |
| 6 | Br | Me | Me | Me | Me | $CH_2CH_2CH_2Cl$ | m.p. = 118-120°C |
| 7 | Br | Me | Me | Me | Et | H | m.p. = 116-118°C |
| 8 | Br | Me | Me | Me | Et | Me | m.p. = 131-133°C |
| 12 | Br | Me | Me | Me | iPr | H | resin; $(M+H)^+ = 435$ |
| 17 | Cl | Me | Me | Me | Me | H | m.p. = 103-105°C |
| 18 | Cl | Me | Me | Me | Me | Me | m.p. = 133-134°C |
| 19 | Cl | Me | Me | Me | Me | $CH_2OMe$ | m.p. = 129-131°C |
| 20 | Cl | Me | Me | Me | Me | Et | m.p. = 134-136°C |
| 21 | Cl | Me | Me | Me | Me | $CH_2CH_2OMe$ | m.p. = 113-114°C |
| 22 | Br | Me | Me | Me | Me | $CH_2OEt$ | m.p. = 99-101°C |

(continued)

| Compound No | Hal | V | $R^1$ | $R^3$ | $R^4$ | $R^5$ | Physical/ spectral data |
|---|---|---|---|---|---|---|---|
| 23 | Br | Me | Me | Me | Me | $CH_2CH(OH)CH_2Cl$ | m.p. = 133-134°C |
| 24 | Br | Me | Me | Me | Me | $CH{=}CHCH_2OH$ | m.p. = 85-87°C |
| 25 | Br | Me | Me | Me | Me | $CH_2CH_2OCH_2CH_2OMe$ | m.p. = 87-90°C |
| 26 | Br | Me | Me | Me | Me | 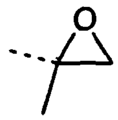 | m.p. = 145-146°C |
| 27 | Br | Me | Me | Me | Me | $C(CH_3)(OH)CH_2Cl$ | m.p. = 138-140°C |
| 28 | Br | Me | Me | Me | Me | $CH_2O$allyl | m.p. = 90-92°C |
| 29 | Br | Me | Me | Me | Me | $CH_2O$propargyl | m.p. = 144-147°C |
| 30 | Br | Me | Me | Me | Me | $CH_2OH$ | m.p. = 137-139°C |
| 31 | Br | Me | Me | Me | Me | $CH_2OCH_2OMe$ | m.p. = 104-105°C |
| 32 | Br | Me | Me | Me | Me | $CH_2OCH_2O(CH_2)_2OMe$ | m.p. = 99-102°C |
| 33 | Br | Et | Me | Me | Me | H | m.p. = 128-130°C |
| 34 | Br | Et | Me | Me | Me | Me | m.p. = 104-106°C |
| 35 | Br | Et | Me | Me | Me | $CH_2OMe$ | m.p. = 112-115°C |
| 36 | Br | Et | Me | Me | Me | $CH_2OEt$ | m.p. = 90-92°C |

The compounds of general formula (1) may be prepared as outlined in Schemes 1 to 4 below, in which Ar, $R^1$, $R^3$, $R^4$ and $R^5$ have the meanings given above, $R^6$ is H or $-C_{1-4}$ alkyl, as indicated (see Scheme 1 below); DMF is *N, N*-dimethylformamide, NBS is *N*-bromosuccinimide, NCS is *N*-chlorosuccinimide and MCPBA is *m*-chioroperbenzoic acid. Other abbreviations are defined in the text.

Compounds of formula (1), where n is 0, may be prepared as shown in Scheme 1. Esters of formula (2), where $R^6$ is $C_{1-4}$ alkyl, may be halogenated to give haloesters of formula (3), where Hal is a halogen atom such as bromine, chlorine or iodine, by reaction with a halogenating agent such as *N*-bromosuccinimide, in a suitable solvent such as carbon tetrachloride or acetonitrile, in the presence of a radical initiator such as AIBN (azo-*iso*butyronitrile), and a light source, at between ambient temperature and the reflux temperature of the solvent. Compounds of general formula (3) are then reacted with alkanethiols of general formula $R^1SH$, in the presence of a base such as sodium hydride, in a suitable solvent such as DMF, to give compounds of general formula (6), or are reacted with alkanethiol salts $R^1S^-M^+$, where M is a metal such as sodium or lithium, in a suitable solvent such as DMF, to give compounds of general formula (6).

Scheme 1

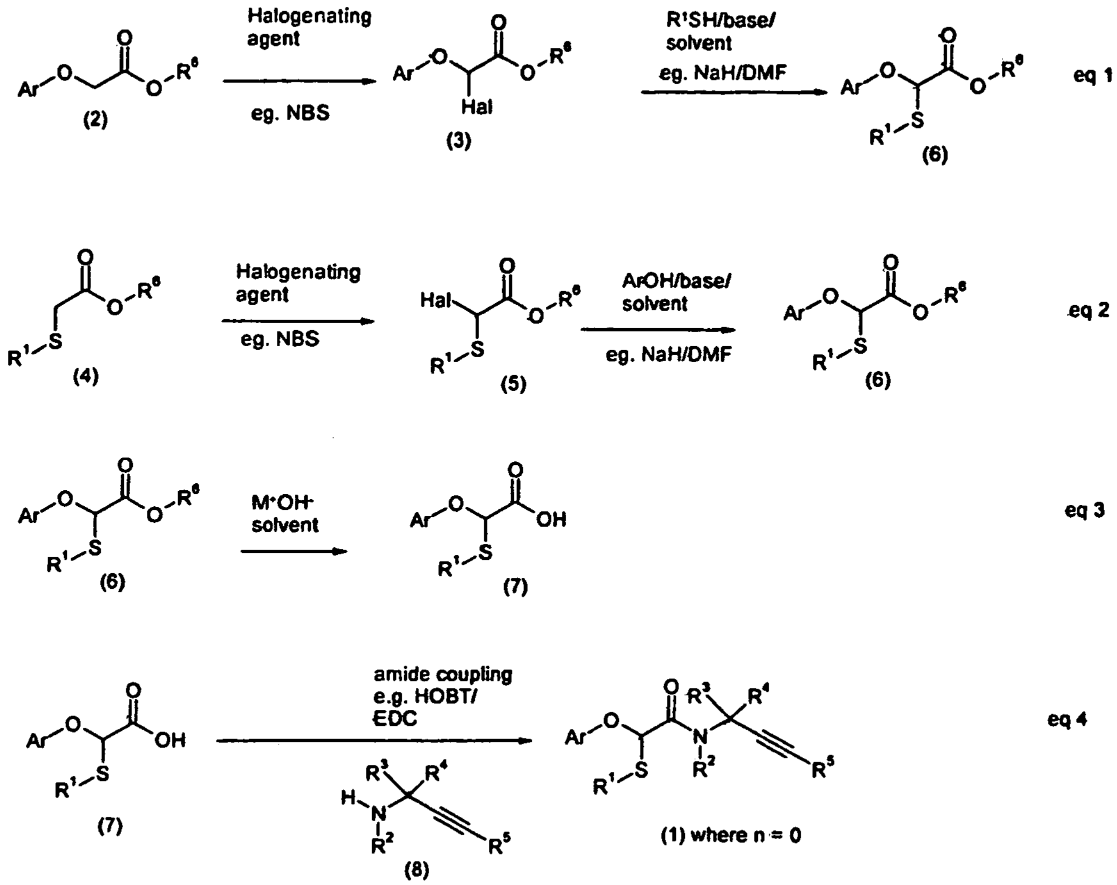


Alternatively esters of general formula (4) are halogenated to give haloesters of formula (5), where Hal is a halogen atom such as bromine, chlorine or iodine, by reaction with a halogenating agent such as *N*-chlorosuccinimide or *N*-bromosuccinimide, in a suitable solvent such as carbon tetrachloride or acetonitrile, at between 0°C and the reflux temperature of the solvent. Haloesters of formula (5) are reacted with hydroxy(hetero)aryls ArOH, where Ar is as defined above, in the presence of a base such as potassium t-butoxide, potassium carbonate, or sodium hydride in a suitable solvent such as t-butanol, 1,4-dioxane or DMF, at between ambient temperature and the reflux temperature of the solvent, to give compounds of formula (6). Compounds of formula (6) are hydrolysed to acids of formula (7) by reaction with an alkali metal hydroxide $M^+OH^-$, in a suitable solvent such as aqueous methanol, ethanol, or THF (tetrahydrofuran) at between ambient temperature and the reflux temperature of the solvent. Acids of formula (7) can be condensed with amines of formula (8), using suitable activating agents such as HOBT (1-hydroxybenzotriazole) and EDC (1-ethyl-3-*N,N*-dimethylaminopropylcarbodiimide hydrochloride), at between 0°C and ambient temperature, to give compounds of general formula (1) where n is 0.

Compounds of general formula (1), where n is 1 or 2, are prepared by oxidation to the sulphoxide (n is 1) or sulphone (n is 2) oxidation state, as shown in Scheme 2. For example, esters of the general formula (6) can be oxidised to sulphoxides of formula (9) with an oxidising agent such as sodium periodate in a suitable solvent such ethanol, between 0°C and ambient temperature. Sulphones of formula (10) can be made either directly from compounds of formula (6) with two or more equivalents of an oxidising agent such as *m*-chloroperbenzoic acid (MCPBA), in a suitable solvent such as dichloromethane between 0°C and the reflux temperature of the solvent, or from sulphoxides of formula (9) with one or more equivalents of *m*-chloroperbenzoic acid. Sulphides of formula (6), sulphoxides of formula (9) or sulphones of formula (10) can be hydrolysed to the corresponding acids (7), (11) or (12) by reaction with an alkali metal hydroxide in a suitable solvent such as ethanol at between 0°C and the reflux temperature of the solvent followed by acidification. The acids of formula (7), (11) or (12) can be condensed with amines of formula (8), wherein $R^2$ is hydrogen, using suitable activating agents such as HOBT and EDC, at between 0°C and ambient temperature, to give compounds of general formula (1) where n is 0, 1 or 2.

## Scheme 2:

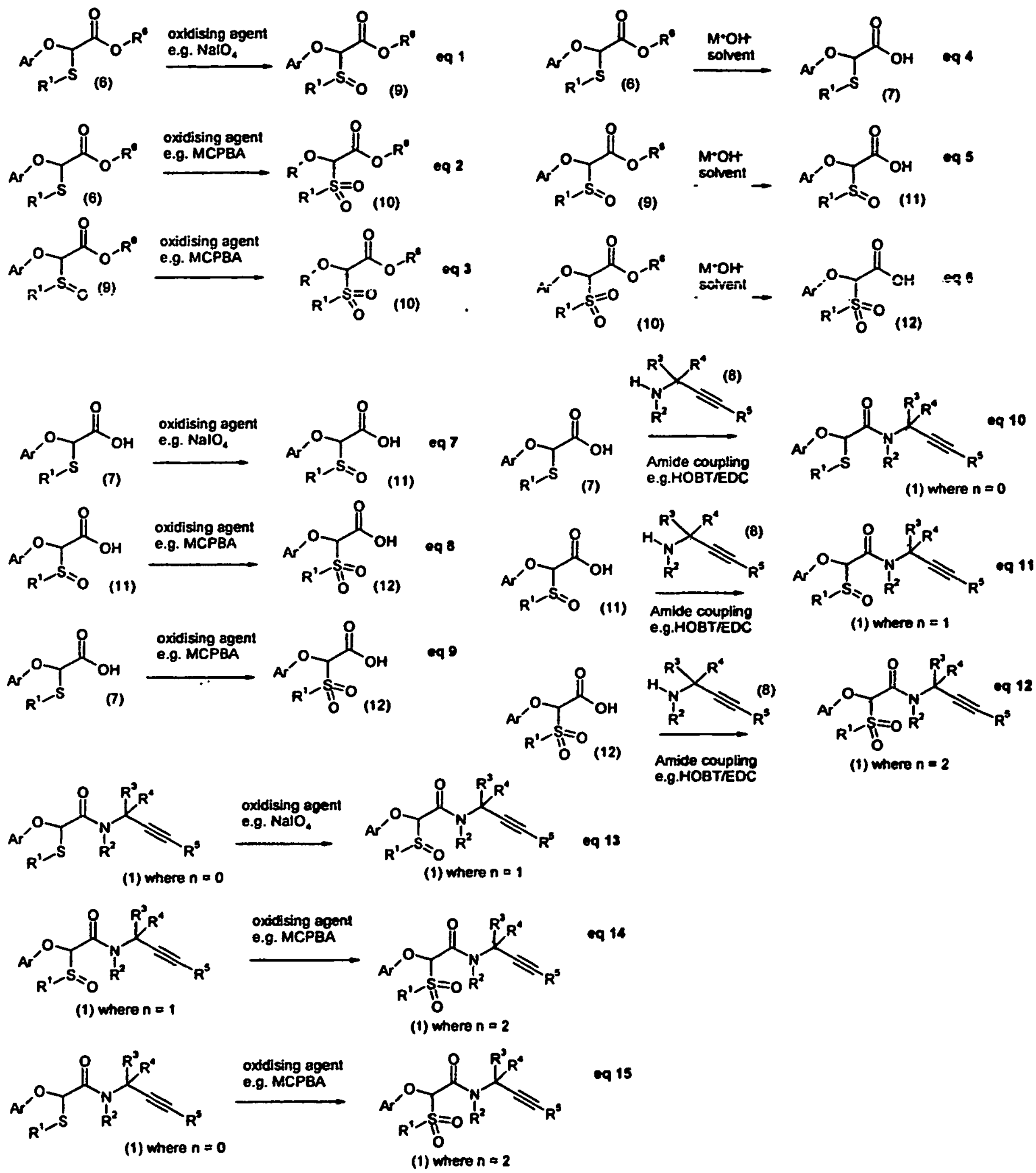


Similarly, sulphoxides of formula (11) and of formula (1) where n is 1 and $R^2$ is hydrogen can be prepared from sulphides of formula (7) and of formula (1) where n is 0 respectively, using oxidising agents such as sodium metaperiodate or m-chloroperbenzoic acid as described above. Sulphones of formula (12) and of formula (1) where n is 2, can be prepared either from sulphides of formula (7) and of formula (1) where n is 0, by using at least two equivalents of oxidising agents such as *m*-chloroperbenzoic acid, or from sulphoxides of formula (11) and of formula (1) where n is 1, using one or more equivalents of oxidising agents such as *m*-chloroperbenzoic acid, as described above.

Compounds of formula (1) can also be prepared as shown in Scheme 3. Acids of formula (13) can be condensed with amines of formula (8), using suitable activating agents such as HOBT and EDC, at between 0°C and ambient temperature, to give compounds of formula (14). Compounds of formula (14) can be halogenated to compounds of

formula (16) using a halogenating agent such as *N*-chlorosuccinimide, in a suitable solvent such as carbon tetrachloride or acetonitrile, at between 0°C and ambient temperature. Amides of formula (16) can also be prepared from acid halides of formula (15) by reaction with amines of formula (8) in the presence of a base such as triethylamine in a suitable solvent such as dichloromethane, at between 0°C and ambient temperature.

Scheme 3

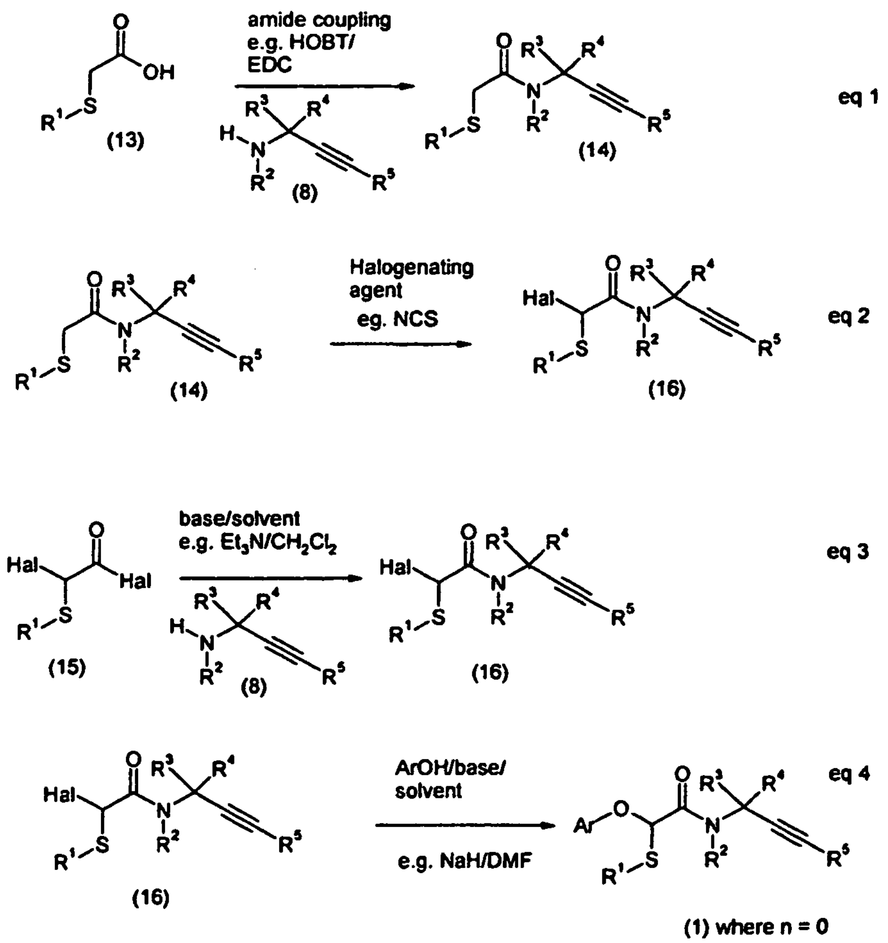


Halosulphides of formula (16) can be reacted with hydroxy (hetero)aryls ArOH, in the presence of a base such as potassium carbonate or sodium hydride, in a suitable solvent such as DMF, at between 0°C and 80°C, to give compounds of formula (1) where n is 0.

As shown in Scheme 4, amines of the general formula (20), which are examples of amines of the general formula (8) wherein $R^2$ is H, may be prepared by alkylation of a silyl-protected aminoalkyne of the general formula (18) using a suitable base, such as n-butyl lithium, followed by reaction with a suitable alkylating reagent $R^5LG$, such as an alkyl iodide, for example, methyl iodide, to form an alkylated compound of the general formula (19). In a similar procedure, a silyl-protected aminoalkyne of the general formula (18) may be reacted with a carbonyl derivative $R^aCOR^b$, for example formaldehyde, using a suitable base, such as n-butyl lithium, to provide an aminoalkyne (19) containing a hydroxyalkyl moiety. The silyl protecting group may then be removed from a compound of the general formula (19) with, for example, an aqueous acid to form an aminoalkyne of the general formula (20). Aminoalkynes of the general formula (20) may be further derivatised, for instance when $R^5$ is a hydroxyalkyl group, for example, by reacting a compound of the general formula (20) with a silylating agent, for example t-butyldimethylsilyl chloride, to give a derivative silylated on oxygen of the general formula (21). In addition, a compound of the general formula (20) may be treated with a base, such as sodium hydride or potassium bis(trimethylsilyl)amide followed by a compound $R^cLG$, to give a compound of the general formula (22). In an alternative sequence, a compound of general formula (19) may be treated with a base, such as sodium or potassium *bis*(trimethylsilyl)amide, followed by a compound $R^cLG$, where LG represents a leaving group such as a halogen, or sulphonate ester such as $OSO_2Me$, or $OSO_2$-4-tolyl, for example ethyl iodide, to give, after removal of the

silyl protecting group, compounds of general formula (22).

## Scheme 4

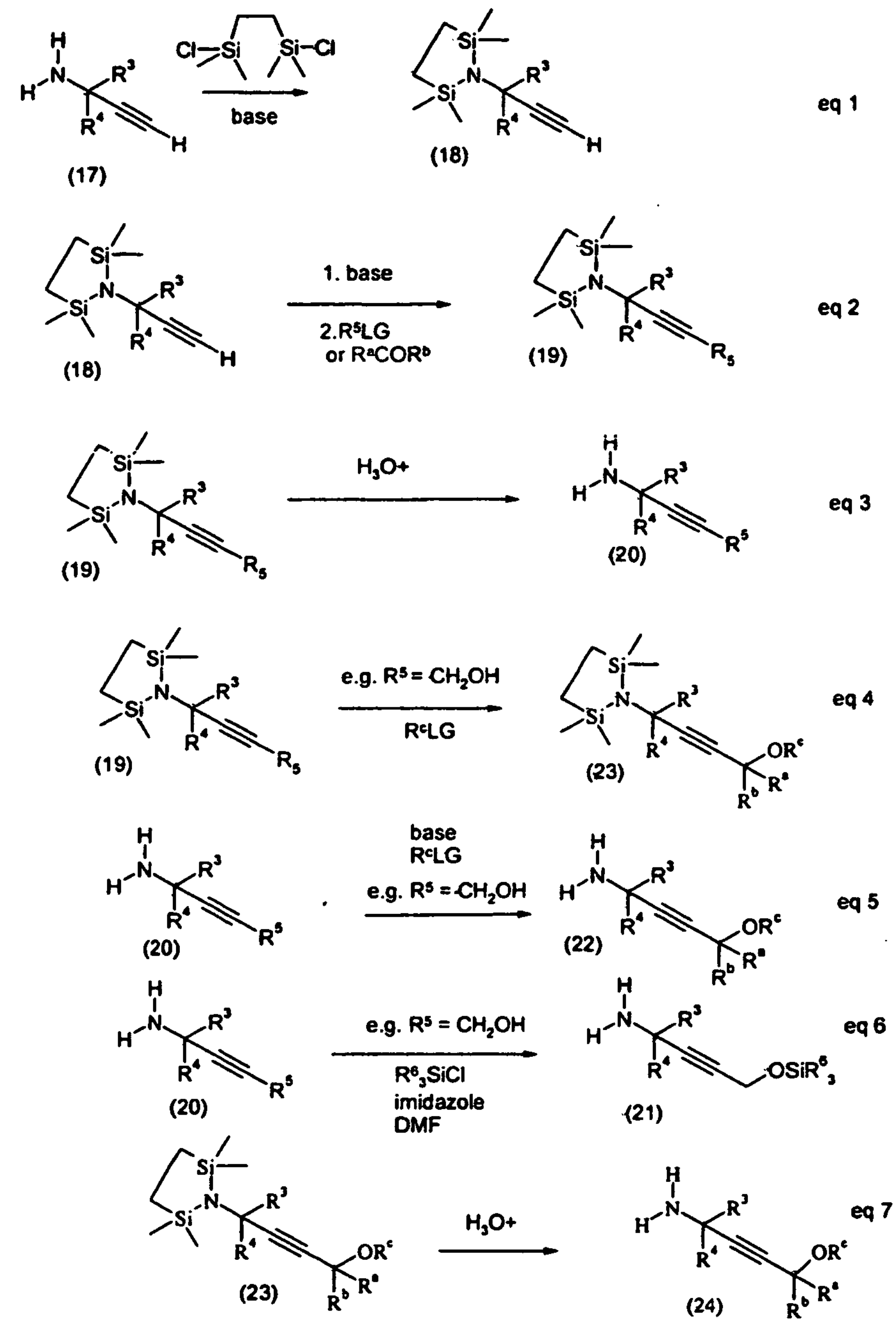


Silyl-protected aminoalkynes of the general formula (18) may be obtained by reacting amines of general formula (17) with 1,2-*bis*-(chlorodimethylsilyl)ethane in the presence of a suitable base, such as a tertiary organic amine base, for example, triethylamine.

Amines of the general formula (17) are either commercially available or may be prepared by standard literature methods (see, for example, EP-A-0834498).

The compounds of formula (1) are active fungicides and may be used to control one or more of the following pathogens: *Pyricularia oryzae (Magnaporthe grisea)* on rice and wheat and other *Pyricularia* spp. on other hosts; *Puccinia triticina* (or *recondita), Puccinia striiformis* and other rusts on wheat, *Puccinia hordei, Puccinia striiformis* and other rusts on barley, and rusts on other hosts (for example turf, rye, coffee, pears, apples, peanuts, sugar beet, vegetables and ornamental plants); *Erysiphe cichoracearum* on cucurbits (for example melon); *Blumeria (or Erysiphe) graminis* (powdery mildew) on barley, wheat, rye and turf and other powdery mildews on various hosts, such as *Sphaerotheca macularis*

on hops, *Sphaerotheca fusca (Sphaerotheca fuliginea)* on cucurbits (for example cucumber), *Leveillula taurica* on tomatoes, aubergine and green pepper, *Podosphaera leucotricha* on apples and *Uncinula necator* on vines; *Cochliobolus* spp., *Helminthosporium* spp., *Drechslera* spp. *(Pyrenophora* spp.), *Rhynchosporium* spp., *Mycosphaerella graminicola (Septoria tritici)* and *Phaeosphaeria nodorum (Stagonospora nodorum* or *Septoria nodorum), Pseudocercosporella herpotrichoides* and *Gaeumannomyces graminis* on cereals (for example wheat, barley, rye), turf and other hosts; *Cercospora arachidicola* and *Cercosporidium personatum* on peanuts and other *Cercospora* spp. on other hosts, for example sugar beet, bananas, soya beans and rice; *Botrytis cinerea* (grey mould) on tomatoes, strawberries, vegetables, vines and other hosts and other *Botrytis* spp. on other hosts; *Alternaria* spp. on vegetables (for example carrots), oil-seed rape, apples, tomatoes, potatoes, cereals (for example wheat) and other hosts; *Venturia* spp. (including *Venturia inaequalis* (scab)) on apples, pears, stone fruit, tree nuts and other hosts; *Cladosporium* spp. on a range of hosts including cereals (for example wheat) and tomatoes; *Monilinia* spp. on stone fruit, tree nuts and other hosts; *Didymella* spp. on tomatoes, turf, wheat, cucurbits and other hosts; *Phoma* spp. on oil-seed rape, turf, rice, potatoes, wheat and other hosts; *Aspergillus* spp. and *Aureobasidium* spp. on wheat, lumber and other hosts; *Ascochyta* spp. on peas, wheat, barley and other hosts; *Stemphylium* spp. *(Pleospora* spp.) on apples, pears, onions and other hosts; summer diseases (for example bitter rot *(Glomerella cingulata),* black rot or frogeye leaf spot *(Botryosphaeria obtusa),* Brooks fruit spot *(Mycosphaerella pomi),* Cedar apple rust *(Gymnosporangium juniperi-virginianae),* sooty blotch *(Gloeodes pomigena),* flyspeck *(Schizothyrium pomi)* and white rot *(Botryosphaeria dothidea))* on apples and pears; *Plasmopara viticola* on vines; other downy mildews, such as *Bremia lactucae* on lettuce, *Peronospora* spp. on soybeans, tobacco, onions and other hosts, *Pseudoperonospora humuli* on hops and *Pseudoperonospora cubensis* on cucurbits; *Pythium* spp. (including *Pythium ultimum*) on turf and other hosts; *Phytophthora infestans* on potatoes and tomatoes and other *Phytophthora* spp. on vegetables, strawberries, avocado, pepper, ornamentals, tobacco, cocoa and other hosts; *Thanatephorus cucumeris* on rice and turf and other *Rhizoctonia* spp. on various hosts such as wheat and barley, peanuts, vegetables, cotton and turf; *Sclerotinia* spp. on turf, peanuts, potatoes, oil-seed rape and other hosts; *Sclerotium* spp. on turf, peanuts and other hosts; *Gibberella fujikuroi* on rice; *Colletotrichum* spp. on a range of hosts including turf, coffee and vegetables; *Laetisaria fuciformis* on turf; *Mycosphaerella* spp. on bananas, peanuts, citrus, pecans, papaya and other hosts; *Diaporthe* spp. on citrus, soybean, melon, pears, lupin and other hosts; *Elsinoe* spp. on citrus, vines, olives, pecans, roses and other hosts; *Verticillium* spp. on a range of hosts including hops, rotatoes and tomatoes; *Pyrenopeziza* spp. on oil-seed rape and other hosts; *Oncobasidium theobromae* on cocoa causing vascular streak dieback; *Fusarium* spp., *Typhula* spp., *Microdochium nivale, Ustilago* spp., *Urocystis* spp., *Tilletia* spp. and *Claviceps purpurea* on a variety of hosts but particularly wheat, barley, turf and maize; *Ramularia* spp. on sugar beet, barley and other hosts; post-harvest diseases particularly of fruit (for example *Penicillium digitatum, Penicillium italicum* and *Trichoderma viride* on oranges, *Colletotrichum musae* and *Gloeosporium musarum* on bananas and *Botrytis cinerea* on grapes); other pathogens on vines, notably *Eutypa lata, -Guignardia bidwellii, Phellinus igniarus, Phomopsis viticola, Pseudopeziza tracheiphila* and *Stereum hirsutum;* other pathogens on trees (for example *Lophodermium seditiosum)* or lumber, notably *Cephaloascus fragrans, Ceratocystis* spp., *Ophiostoma piceae, Penicillium* spp., *Trichoderma pseudokoningii, Trichoderma viride, Trichoderma harzianum, Aspergillus niger, Leptographium lindbergi* and *Aureobasidium pullulans;* and fungal vectors of viral diseases (for example *Polymyxa graminis* on cereals as the vector of barley yellow mosaic virus (BYMV) and *Polymyxa betae* on sugar beet as the vector of rhizomania).

The compounds of formula (1) show particularly good activity against the Oomycete class of pathogens such as *Phytophthora infestans, Plasmopara* species, e.g. *Plasmopara viticola* and *Pythium* species e.g. *Pythium ultimum.*

A compound of formula (1) may move acropetally, basipetally or locally in plant tissue to be active against one or more fungi. Moreover, a compound of formula (1) may be volatile enough to be active in the vapour phase against one or more fungi on the plant.

The invention therefore provides a method of combating or controlling phytopathogenic fungi which comprises applying a fungicidally effective amount of a compound of formula (1), or a composition containing a compound of formula (1), to a plant, to a seed of a plant, to the locus of the plant or seed or to soil or any other plant growth medium, e.g. nutrient solution.

The term "plant" as used herein includes seedlings, bushes and trees. Furthermore, the fungicidal method of the invention includes protectant, curative, systemic, eradicant and antisporulant treatments.

The compounds of formula (1) are preferably used for agricultural, horticultural and turfgrass purposes in the form of a composition.

In order to apply a compound of formula (1) to a plant, to a seed of a plant, to the locus of the plant or seed or to soil or any other growth medium, a compound of formula (1) is usually formulated into a composition which includes, in addition to the compound of formula (1), a suitable inert diluent or carrier and, optionally, a surface active agent (SFA). SFAs are chemicals that are able to modify the properties of an interface (for example, liquid/solid, liquid/air or liquid/liquid interfaces) by lowering the interfacial tension and thereby leading to changes in other properties (for example dispersion, emulsification and wetting). It is preferred that all compositions (both solid and liquid formulations) comprise, by weight, 0.0001 to 95%, more preferably 1 to 85%, for example 5 to 60%, of a compound of formula (1). The composition

is generally used for the control of fungi such that a compound of formula (1) is applied at a rate of from 0.1g to 10kg per hectare, preferably from 1g to 6kg per hectare, more preferably from 1g to 1kg per hectare.

When used in a seed dressing, a compound of formula (1) is used at a rate of 0.0001g to 10g (for example 0.001g or 0.05g), preferably 0.005g to 10g, more preferably 0.005g to 4g, per kilogram of seed.

In another aspect the present invention provides a fungicidal composition comprising a fungicidally effective amount of a compound of formula (1) and a suitable carrier or diluent therefor.

In a still further aspect the invention provides a method of combating and controlling fungi at a locus, which comprises treating the fungi, or the locus of the fungi with a fungicidally effective amount of a composition comprising a compound of formula (1). The compositions can be chosen from a number of formulation types, including dustable powders (DP), soluble powders (SP), water soluble granules (SG), water dispersible granules (WG), wettable powders (WP), granules (GR) (slow or fast release), soluble concentrates (SL), oil miscible liquids (OL), ultra low volume liquids (UL), emulsifiable concentrates (EC), dispersible concentrates (DC), emulsions (both oil in water (EW) and water in oil (EO)), micro-emulsions (ME), suspension concentrates (SC), aerosols, fogging/smoke formulations, capsule suspensions (CS) and seed treatment formulations. The formulation type chosen in any instance will depend upon the particular purpose envisaged and the physical, chemical and biological properties of the compound of formula (1).

Dustable powders (DP) may be prepared by mixing a compound of formula (1) with one or more solid diluents (for example natural clays, kaolin, pyrophyllite, bentonite, alumina, montmorillonite, kieselguhr, chalk, diatomaceous earths, calcium phosphates, calcium and magnesium carbonates, sulphur, lime, flours, talc and other organic and inorganic solid carriers) and mechanically grinding the mixture to a fine powder.

Soluble powders (SP) may be prepared by mixing a compound of formula (1) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of formula (1) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of formula (1) and one or more powdered solid diluents or carriers, or from pre-formed blank granules by absorbing a compound of formula (1) (or a solution thereof, in a suitable agent) in a porous granular material (such as pumice, attapulgite clays, fuller's earth, kieselguhr, diatomaceous earths or ground corn cobs) or by adsorbing a compound of formula (1) (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of formula (1) in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of formula (1) in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone), alcohols (such as benzyl alcohol, furfuryl alcohol or butanol), *N*-alkylpyrrolidones (such as *N*-methylpyrrolidone or N-octyl-pyrrolidone), dimethyl amides of fatty acids (such as $C_8$-$C_{10}$ fatty acid dimethylamide) and chlorinated hydrocarbons. An EC product may spontaneously emulsify on addition to water, to produce an emulsion with sufficient stability to allow spray application through appropriate equipment. Preparation of an EW involves obtaining a compound of formula (1) either as a liquid (if it is not a liquid at ambient temperature, it may be melted at a reasonable temperature, typically below 70°C) or in solution (by dissolving it in an appropriate solvent) and then emulsifying the resultant liquid or solution into water containing one or more SFAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents that have a low solubility in water.

Microemulsions (ME) may be prepared by mixing water with a blend of one or more solvents with one or more SFAs, to produce spontaneously a thermodynamically stable isotropic liquid formulation. A compound of formula (1) is present initially in either the water or the solvent/SFA blend. Suitable solvents for use in MEs include those hereinbefore described for use in in ECs or in EWs. An ME may be either an oil-in-water or a water-in-oil system (which system is

present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely dividend insoluble solid particles of a compound of formula (1). SCs maybe prepared by ball or bead milling the solid compound of formula (1) in a suitable medium, optionally with one or more dispersing agents, to produce a fine particle suspension of the compound. One or more wetting agents may be included in the composition and a suspending agent may be included to reduce the rate at which the particles settle. Alternatively, a compound of formula (1) may be dry milled and added to water, containing agents hereinbefore described, to produce the desired end product.

Aerosol formulations comprise a compound of formula (1) and a suitable propellant (for example n-butane). A compound of formula (1) may also be dissolved or dispersed in a suitable medium (for example water or a water miscible liquid, such as n-propanol) to provide compositions for use in non-pressurised, hand-actuated spray pumps.

A compound of formula (1) may be mixed in the dry state with a pyrotechnic mixture to form a composition suitable for generating, in an enclosed space, a smoke containing the compound.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of formula (1) and, optionally, a carrier or diluent therefor. The polymeric shell may be produced by either an interfacial polycondensation reaction or by a coacervation procedure. The compositions may provide for controlled release of the compound of formula (1) and they may be used for seed treatment. A compound of formula (1) may also be formulated in a biodegradable polymeric matrix to provide a slow, controlled release of the compound.

A composition may include one or more additives to improve the biological performance of the composition (for example by improving wetting, retention or distribution on surfaces; resistance to rain on treated surfaces; or uptake or mobility of a compound of formula (1)). Such additives include surface active agents, spray additives based on oils, for example certain mineral oils or natural plant oils (such as soy bean and rape seed oil), and blends of these with other bio-enhancing adjuvants (ingredients which may aid or modify the action of a compound of formula (1)).

A compound of formula (1) may also be formulated for use as a seed treatment, for example as a powder composition, including a powder for dry seed treatment (DS), a water soluble powder (SS) or a water dispersible powder for slurry treatment (WS), or as a liquid composition, including a flowable concentrate (FS), a solution (LS) or a capsule suspension (CS). The preparations of DS, SS, WS, FS and LS compositions are very similar to those of, respectively, DP, SP, WP, SC and DC compositions described above. Compositions for treating seed may include an agent for assisting the adhesion of the composition to the seed (for example a mineral oil or a film-forming barrier).

Wetting agents, dispersing agents and emulsifying agents may be SFAs of the cationic, anionic, amphoteric or non-ionic type.

Suitable SFAs of the cationic type include quaternary ammonium compounds (for example cetyltrimethyl ammonium bromide), imidazolines and amine salts. Suitable anionic SFAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butylnaphthalene sulphonate and mixtures of sodium diisopropyl- and tri-*iso*propyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono esters) or phosphorus pentoxide (predominately di-esters), for example the reaction between lauryl alcohol and tetraphosphoric acid; additionally these products may be ethoxylated), sulphosuccinamates, paraffin or olefine sulphonates, taurates and lignosulphonates.

Suitable SFAs of the amphoteric type include betaines, propionates and glycinates.

Suitable SFAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); and lecithins.

Suitable suspending agents include hydrophilic colloids (such as polysaccharides, polyvinylpyrrolidone or sodium carboxymethylcellulose) and swelling clays (such as bentonite or attapulgite).

A compound of formula (1) may be applied by any of the known means of applying fungicidal compounds. For example, it may be applied, formulated or unformulated, to any part of the plant, including the foliage, stems, branches or roots, to the seed before it is planted or to other media in which plants are growing or are to be planted (such as soil surrounding the roots, the soil generally, paddy water or hydroponic culture systems), directly or it may be sprayed on, dusted on, applied by dipping, applied as a cream or paste formulation, applied as a vapour or applied through distribution

or incorporation of a composition (such as a granular composition or a composition packed in a water-soluble bag) in soil or an aqueous environment.

A compound of formula (1) may also be injected into plants or sprayed onto vegetation using electrodynamic spraying techniques or other low volume methods, or applied by land or aerial irrigation systems.

Compositions for use as aqueous preparations (aqueous solutions or dispersions) are generally supplied in the form of a concentrate containing a high proportion of the active ingredient, the concentrate being added to water before use. These concentrates, which may include DCs, SCs, ECs, EWs, MEs SGs, SPs, WPs, WGs and CSs, are often required to withstand storage for prolonged periods and, after such storage, to be capable of addition to water to form aqueous preparations which remain homogeneous for a sufficient time to enable them to be applied by conventional spray equipment. Such aqueous preparations may contain varying amounts of a compound of formula (1) (for example 0.0001 to 10%, by weight) depending upon the purpose for which they are to be used.

A compound of formula (1) may be used in mixtures with fertilisers (for example nitrogen-, potassium- or phosphorus-containing fertilisers). Suitable formulation types include granules of fertiliser. The mixtures suitably contain up to 25% by weight of the compound of formula (1).

The invention therefore also provides a fertiliser composition comprising a fertiliser and a compound of formula (1).

The compositions of this invention may contain other compounds having biological activity, for example micronutrients or compounds having similar or complementary fungicidal activity or which possess plant growth regulating, herbicidal, insecticidal, nematicidal or acaricidal activity.

By including another fungicide, the resulting composition may have a broader spectrum of activity or a greater level of intrinsic activity than the compound of formula (1) alone. Further the other fungicide may have a synergistic effect on the fungicidal activity of the compound of formula (1).

The compound of formula (1) may be the sole active ingredient of the composition or it may be admixed with one or more additional active ingredients such as a pesticide, fungicide, synergist, herbicide or plant growth regulator where appropriate. An additional active ingredient may: provide a composition having a broader spectrum of activity or increased persistence at a locus; synergise the activity or complement the activity (for example by increasing the speed of effect or overcoming repellency) of the compound of formula (1); or help to overcome or prevent the development of resistance to individual components. The particular additional active ingredient will depend upon the intended utility of the composition.

Examples of fungicidal compounds which may be included in the composition of the invention are AC 382042 (*N*-(1-cyano-1,2-dimethylpropyl)-2-(2,4-dichlorophenoxy) pro-pionamide), acibenzolar-S-methyl, alanycarb, aldimorph, anilazine, azaconazole, azafenidin, azoxystrobin, benalaxyl, benomyl, benthiavalicarb, biloxazol, bitertanol, blasticidin S, boscalid (new name for nicobifen), bromuconazole, bupirimate, captafol, captan, carbendazim, carbendazim chlorhydrate, carboxin, carpropamid, carvone, CGA 41396, CGA 41397, chinomethionate, chlorbenzthiazone, chlorothalonil, chlorozolinate, clozylacon, copper containing compounds such as copper oxychloride, copper oxyquinolate, copper sulphate, copper tallate, and Bordeaux mixture, cyamidazosulfamid, cyazofamid (IKF-916), cyflufenamid, cymoxanil, cyproconazole, cyprodinil, debacarb, di-2-pyridyl disulphide 1,1'-dioxide, dichlofluanid, diclocymet, diclomezine, dicloran, diethofencarb, difenoconazole, difenzoquat, diflumetorim, *O,O*-di-*iso*-propyl-S-benzyl thiophosphate, dimefluazole, dimetconazole, dimethirimol, dimethomorph, dimoxystrobin, diniconazole, dinocap, dithianon, dodecyl dimethyl ammonium chloride, dodemorph, dodine, doguadine, edifenphos, epoxiconazole, ethaboxam, ethirimol, ethyl (*Z*)-*N*-benzyl-*N*([methyl-(methyl-thioethylideneaminooxy-carbonyl)amino]thio)-β-alaninate, etridiazole, famoxadone, fenamidone, fenarimol, fenbuconazole, fenfuram, fenhexamid, fenoxanil (AC 382042), fenpiclonil, fenpropidin, fenpropimorph, fentin acetate, fentin hydroxide, ferbam, ferimzone, fluazinam, fludioxonil, flumetover, flumorph, fluoroimide, fluoxastrobin, fluquinconazole, flusilazole, flusulfamide, flutolanil, flutriafol, folpet, fosetyl-aluminium, fuberidazole, furalaxyl, furametpyr, guazatine, hexaconazole, hydroxyisoxazole, hymexazole, imazalil, imibenconazole, iminoctadine, iminoctadine triacetate, ipconazole, iprobenfos, iprodione, iprovalicarb, isopropanyl butyl carbamate, isoprothiolane, kasugamycin, kresoxim-methyl, LY186054, LY211795, LY 248908, mancozeb, maneb, mefenoxam, mepanipyrim, mepronil, metalaxyl, metalaxyl M, metconazole, metiram, metiram-zinc, metominostrobin, metrafenone, MON65500 (*N*-allyl-4,5-dimethyl-2-trimethylsilylthiophene-3-carboxamide), myclobutanil, NTN0301, neoasozin, nickel dimethyldithiocarbamate, nitrothale-isopropyl, nuarimol, ofurace, organomercury compounds, orysastrobin, oxadixyl, oxasulfuron, oxolinic acid, oxpoconazole, oxycarboxin, pefurazoate, penconazole, pencycuron, phenazin oxide, phosphorus acids, phthalide, picoxystrobin, polyoxin D, polyram, probenazole, prochloraz, procymidone, propamocarb, propamocarb hydrochloride, propiconazole, propineb, propionic acid, proquinazid, prothioconazole, pyraclostrobin, pyrazophos, pyrifenox, pyrimethanil, pyroquilon, pyroxyfur, pyrrolnitrin, quaternary ammonium compounds, quinomethionate, quinoxyfen, quintozene, silthiofam (MON 65500), S-imazalil, simeconazole, sipconazole, sodium pentachlorophenate, spiroxamine, streptomycin, sulphur, tebuconazole, tecloftalam, tecnazene, tetraconazole, thiabendazole, thifluzamide, 2-(thiocyano-methylthio)benzothiazole, thiophanate-methyl, thiram, tiadinil, timibenconazole, tolclofos-methyl, tolylfluanid, triadimefon, triadimenol, triazbutil, triazoxide, tricyclazole, tridemorph, trifloxystrobin, triflumizole, triforine, triticonazole, validamycin A, vapam, vinclozolin,

XRD-563, zineb, ziram, zoxamide and compounds of the formulae:

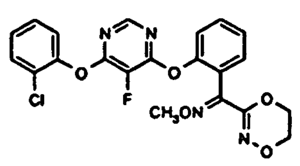
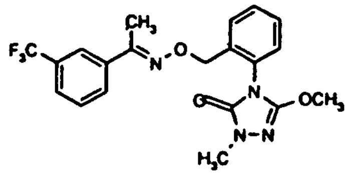
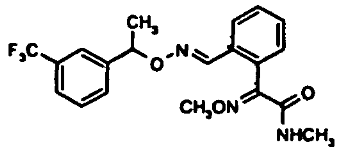

The compounds of formula (1) may be mixed with soil, peat or other rooting media for the protection of plants against seed-borne, soil-borne or foliar fungal diseases.

Some mixtures may comprise active ingredients, which have significantly different physical, chemical or biological properties such that they do not easily lend themselves to the same conventional formulation type. In these circumstances other formulation types may be prepared. For example, where one active ingredient is a water insoluble solid and the other a water insoluble liquid, it may nevertheless be possible to disperse each active ingredient in the same continuous aqueous phase by dispersing the solid active ingredient as a suspension (using a preparation analogous to that of an SC) but dispersing the liquid active ingredient as an emulsion (using a preparation analogous to that of an EW). The resultant composition is a suspoemulsion (SE) formulation.

The invention is illustrated by the following Examples.

EXAMPLE 1

This Example illustrates the preparation of 2-(3-Chloro-8-methyl-quinolin-6-yloxy)-N-(1,1-dimethyl-prop-2-ynyl)-2-methylsulfanyl-acetamide (compound No.1 of Table 1).

Stage 1: Preparation of 3-Chloro-8-methyl-quinolin-6-ol

Step 1: Preparation of 8-Methyl-6-nitro-quinoline

3-Nitro-benzenesulfonic acid sodium salt (78.8 g) and propane-1,2,3-triol (108.95 g) are added at ambient temperature to a freshly prepared solution of sulphuric acid (95-97%) (193.4 g) in water (82.9 g). The resulting solution is stirred at 100°C and 2-Methyl-4-nitro aniline (50.0 g) is added. The reaction mixture is further stirred at 150°C for 4 hrs. The reaction mixture is cooled down to ambient temperature then treated with water, carefully neutralized with powdered $NaHCO_3$ and, extracted with ethyl acetate. The organic phase is washed with a saturated NaCl solution, dried over sodium sulphate and concentrated under reduced pressure. The residue is purified by flash filtration on silica gel (hexane/ethyl acetate) and further recrystallized in isopropanol to give 8-Methyl-6-nitro-quinoline (27.44 g) as a yellow solid.

$^1$H NMR ($CDCl_3$) $\delta$ ppm: 9.10 (1H, sm); 8.62 (1H, sm); 8.30 (2H, sm); 7.58 (1H, dd), 2.88 (3H, s).

Step 2: Preparation of 3-Chloro-8-methyl-6-nitro-quinoline

8-Methyl-6-nitro-quinoline (8.22 g) and N-Chlorosuccinimide (11.66 g) in water free acetic acid (100 ml) are stirred at 80°C for 48 hrs. The reaction mixture is cooled down to ambient temperature then, treated with water, carefully neutralized with solid (powder) $NaHCO_3$ and further stirred at R.T. for 30 minutes. Powdered sodium thiosulfate is carefully added to remove the excess of N-Chlorosuccinimide. The reaction mixture is stirred at ambient temperature for 2 hrs, extracted with ethyl acetate. The organic phase is washed with a saturated NaCl solution, dried over sodium sulphate and concentrated under reduced pressure. The residue is triturated in *tert-butyl* methyl ether to give 3-Chloro-8-methyl-6-nitro-quinoline (6.36 g) as a white solid.

$^1$H NMR ($CDCl_3$) $\delta$ ppm: 9.00 (1H, sd); 8.54 (1H, sm); 8.31 (1H, sm); 8.27 (1H, sd), 2.88 (3H, s).

Step 3: Preparation of 3-Chloro-8-methyl-quinolin-6-ylamine

At ambient temperature, Iron (17.52 g) is added to a mixture of 3-Chloro-8-methyl-6-nitro-quinoline (10.58 g) in acetic acid/water/ethyl acetate (145 ml/6.3 ml/14.3 ml). The reaction mixture is stirred at 60°C for 2 hrs then, filtrated over celite. Careful addition of aqueous sodium hydroxide solution (2N) brought the pH of the medium to 10. After extraction with ethyl acetate, the organic phase is washed with a saturated NaCl solution, dried over sodium sulphate and concentrated under reduced pressure to give 3-Chloro-8-methyl-quinolin-6-ylamine (9.21 g) as a light brown solid crude residue which is used in the next step without further purification.

$^1H$ NMR ($CDCl_3$) δ ppm: 8.52 (1H, sm); 7.80 (1H, sm); 6.98 (1H, sm); 6.63 (1H, sm), 3.95 (2H, bs); 2.68 (3H, s).

Step 4: Preparation of 3-Chloro-8-methyl-quinolin-6-ol

3-Chloro-8-methyl-quinolin-6-ylamine (12.00 g) in a mixture of phosphoric acid (400 ml) and water (40 ml) are heated at 170°C for 24 hrs in an autoclave (pressure: 1.0-2.7 bars). The reaction mixture is cooled down to ambient temperature then, treated with water (1000 ml) and stirred at ambient temperature for 2 hrs. The precipitate is filtered off to give a first batch of 3-Chloro-8-methyl-quinolin-6-ol (5.39 g + 1.36 g = 6.75 g) as a brown solid. The filtrate is extracted (4x) with ethyl acetate. The organic phase is dried over sodium sulphate and concentrated under reduced pressure to give a second batch of 3-Chloro-8-methyl-quinolin-6-ol (6.41 g) as a light brown solid.
$^1H$ NMR (DMSO $d_6$) δ ppm: 10.1 (1H, bs); 8.61 (1H, sd); 8.29 (1H, sd); 7.18 (1H, sm), 6.96 (1H, sm); 2.62 (3H, s).

Stage 2: Preparation of Chloro-methylsulfanyl-acetic acid ethyl ester

To a stirred solution of ethyl (methylthio)acetate (10.8 ml) in dichloromethane (300ml) cooled to -15°C is added dropwise sulphuryl chloride (8.1 ml). The mixture is allowed to warm to room temperature over two hours and then concentrated under reduced pressure to give crude chloro-methylsulfanyl-acetic acid ethyl ester as a colourless liquid. The product is used in the next step without further purification.
$^1H$ NMR ($CDCl_3$) δ ppm: 5.35 (1H, s); 4.25 (2H, m); 2.30 (3H,s); 1.30 (3H, t).

Stage 3: Preparation of 2-(3-Chloro-8-methyl-quinolin-6-yloxy)-N-(1,1-dimethyl-prop-2-ynyl)-2-methylsulfanyl-acetamide according to Scheme 1

Step 1: Preparation of (3-Chloro-8-methyl-quinolin-6-yloxy)-methylsulfanyl-acetic acid ethyl ester

Chloro-methylsulfanyl-acetic acid ethyl ester (7.03 g) from Example 1, stage 2 above and, dry potassium carbonate (24.02 g) are added to a solution of 3-Chloro-8-methyl-quinolin-6-ol (6.73 g) in N,N-dimethylformamide (50 ml) at ambient temperature. The reaction mixture is stirred at 60°C for 3 hrs then treated with water and extracted with ethyl acetate. The organic phase is washed with a saturated NaCl solution, dried over sodium sulphate and concentrated under reduced pressure. The residue is purified by flash chromatography (hexane/ethyl acetate) to give (3-Chloro-8-methyl-quinolin-6-yloxy)-methylsulfanyl-acetic acid ethyl ester (7.58 g) as a yellow oil.
$^1H$ NMR ($CDCl_3$) δ ppm: 8.70 (1H, sm); 7.98 (1H, sm); 7.34 (1H, sm); 6.95 (1H, sm), 5.68 (1H, s); 4.35 (2H, qd); 2.76 (3H, s); 2.25 (3H, s); 1.34 (3H, t).

Step 2: Preparation of (3-Chloro-8-methyl-quinolin-6-yloxy)-methylsulfanyl-acetic acid

To (3-Chloro-8-methyl-quinolin-6-yloxy) methylsulfanyl acetic acid ethyl ester (7.53 g) in methanol (100 ml) at ambient temperature is added an aqueous solution of sodium hydroxide (2N; 17.33 ml). The reaction mixture is stirred at 70°C for 1 hr and cooled down to ambient temperature and then, ~90% of the solvent is evaporated. The residue is treated with water and the resulting mixture is taken to pH=1 with addition of an aqueous solution of HCl (1 N). The precipitate is filtered off, washed with water and dried under reduced pressure to give (3-Chloro-8-methyl-quinolin-6-yloxy) methylsulfanyl acetic acid (6.69 g) as a yellow solid.
$^1H$ NMR (DMSO $d_6$) δ ppm: 13.5 (1H, bs); 8.73 (1H, sm); 8.34 (1H, sm); 7.42 (1H, sm); 7.29 (1H, sm), 6.04 (1H, s); 2.68 (3H, s); 2.25 (3H, s); 2.17 (3H, s).

Step 3: Preparation of 2-(3-Chloro-8-methyl-quinolin-6-yloxy)-N-(1,1-dimethyl-prop-2-ynyl)-2-methylsulfanyl-acetamide

N-Ethyl diisopropyl amine (0.130 g), (Benzotriazol-1-yloxy)-tris-(dimethylamino)-phosphonium-hexafluorophosphate (0.205 g) and, N,N-dimethyl amino pyridine (cat.) are added sequentially to a stirred solution of (3-Chloro-8-methyl-quinolin-6-yloxy) methylsulfanyl acetic acid (0.120 g) and 1,1-Dimethyl-but-2-ynylamine hydrochloride (0.054 g) in dry DMF (2 ml). The reaction mixture is stirred at ambient temperature for 2 hrs then treated with water and ethyl acetate. After a liquid-liquid extraction, the organic phase is concentrated under reduced pressure and the residue is purified by flah chromatography on silica gel (hexane/ethyl acetate: 7/3) to give 2-(3-Chloro-8-methyl-quinolin-6-yloxy)-N-(1,1-dimethyl-prop-2-ynyl)-2-methylsulfanyl-acetamide (0.150 g) as a pale yellow solid.
$^1H$ NMR ($CDCl_3$) δ ppm: 8.75 (1H, sm); 8.02 (1H, sm); 7.31 (1H, sm); 7.02 (1H, sm), 6.22 (1H, bs); 5.60 (1H, s); 2.78 (3H, s); 2.20 (3H, s); 1.83 (3H, s); 1.69 (3H, s); 1.68 (3H, s).

EXAMPLE 2

This Example illustrates the preparation of 2-(3-Bromo-8-ethyl-quinolin-6-yloxy)-N-(1,1-dimethyl-but-2-ynyl)-2-methylsulfanyl-acetamide (compound No.34 of Table 4).

Stage 1: Preparation of 3-Bromo-8-ethyl-quinolin-6-ol

Step 1: Preparation of 2-ethyl-4-methoxy-1-nitro-benzene

A solution of ethyl magnesium chloride in THF (2.8M, 16.2ml, 45mmol) is added dropwise to a stirred solution of nitroarene (4.5g, 30 mmol) in THF (150 ml) cooled to - 70°C under argon. After 10 min, powdered KMnO4 (7.2g) is added followed by condensed liquid $NH_3$ (ca. 150 ml). The reaction mixture is stirred for 20 min, NH4Cl (318 mg, 6 mmol) is added and the cooling bath is removed. On reaching -30°C, the mixture is stirred 15min and a saturated solution of oxalic acid in aq. HCl (20 ml, 10%) is added. After extraction with $CH_2Cl_2$, the organic layer is dried over $MgSO_4$, filtered and concentrated *in vacuo.* Purification on silica gel by column chromatography using dichloromethane/hexane mixtures provided 1.9g of 2-ethyl-4-methoxy-1-nitro-benzene. $^1$H NMR ($CDCl_3$) $\delta$ ppm: 8.04-8.01 (1H, m); 6.80-6.78 (2H, m); 3.83 (3H, s); 2.97 (2H, q); 1.28 (3H, t).

Step 2: Preparation of 2-ethyl-4-methoxy-phenylamine

To 2-ethyl-4-methoxy-1-nitro-bentene (750 mg) from Step 1 in ethanol (20 ml) is added iron powder (1.6 g) and 37% aqueous HCl (160 $\mu$l). The mixture is stirred for 2h at room temperature after which time 2M NaOH is added to bring the pH to 8. Extraction with ethyl acetate followed by filtration over celite gave 621 mg of 2-ethyl-4-methoxy-phenylamine which is used as such in Step 3 below.
$^1$H NMR ($CDCl_3$) $\delta$ ppm: 6,68 (1H, s); 6.62-6.61 (2H, m); 3.74 (3H, s); 2.50 (2H, q); 1.23 (3H, t).

Step 3: Preparation of 3-bromo-8-ethyl-6-methoxy-quinoline 2-ethyl-4-methoxy-phenylamine (300 mg) from Step 2 in acetic acid (3 ml) is treated with 2,2,3 tribromopropanal (544 mg) and the mixture is stirred at room temperature for 1 h after which it is diluted with water and extracted with ethyl acetate. The organic phase is washed with 2N NaOH, dried over sodium sulphate, filtered and evaporated under reduced pressure to give 216 mg of 3-bromo-8-ethyl-6-methoxy-quinoline after chromatography (silica; hexane/ethyl acetate) ($M^{+2}$ 268).

$^1$H NMR ($CDCl_3$) $\delta$ ppm: 8.74 (1H, d); 8.16 (1H, d); 7.22 (1H, d); 6.81 (1H, d), 3.21 (3H, s); 2.50 (2H, q); 1.35 (3H, t).

Step 4: Preparation of 3-bromo-8-ethyl-quinolin-6-ol

A mixture of the 3-bromo-8-ethyl-6-methoxy-quinoline (190 mg) from Step 3 in dichloromethane (14 ml) and boron tribromide in dichloromethane (1M, 2.25 ml) is stirred at room temperature for 26 hours. The mixture is cooled to 0°C, treated with MeOH and stirred overnight. The mixture is then filtered and the filtrate evaporated under reduced pressure to provide 134mg of 3-bromo-8-ethyl-quinolin-6-ol ($M^{+2}$ 254). $^1$H NMR (DMSO $d_6$) $\delta$ ppm: 8.69 (1H, d); 8.47 (1H, d); 7.21 (1H, d); 6.99 (1H, s), 3.12 (2H, q); 1.26 (3H, t).

Stage 2: Preparation of 2-(3-Bromo-8-ethyl-quinolin-6-yloxy)-N-(1,1-dimethyl-but-2-ynyl)-2-methylsulfanyl-acetamide according to Scheme 1 by analogy to the procedures described in Example 1, stage3, steps 1 to 3; starting from 3-bromo-8-ethyl-quinolin-6-ol and Chloro-methylsulfanyl-acetic acid ethyl ester; m.p. = 104-106°C.

The following amides of the general formula (1) are prepared using similar procedures to those described in Examples 1 and 2; using the corresponding building blocks:

3-Chloro-8-methyl-quinolin-6-ol: prepared as described in Example 1, stage 1;
3-Bromo-8-methyl-quinolin-6-ol: prepared as described in WO 2006058700 A1;
3-Iodo-8-methyl-quinolin-6-ol: prepared as described in WO 2006058700 A1;
3-Bromo-8-ethyl-quinolin-6-ol: prepared as described in Example 2, stage 1;
Chloro-methylsulfanyl-acetic acid ethyl ester: prepared as described in Example 1, stage 2;
1,1-Dialkyl-prop-2-ynylamine hydrochloride derivatives are either known compounds or may be made from commercially available and/or known compounds by those skilled in the art.

Amides of the general formula (1):

2-(3-Bromo-8-methyl-quinolin-6-yloxy)-N-(1,1-dimethyl-prop-2-ynyl)-2-methylsulfanyl-acetamide (Compound No1 of Table 2 and No2 of Table 4);

2-(3-Bromo-8-methyl-quinolin-6-yloxy)-N-(4-methoxy-1,1-dimethyl-but-2-ynyl)-2-methylsulfanyl-acetamide (Compound No23 of Table 2 and No3 of Table 4);
2-(3-Bromo-8-methyl-quinoline-6-yloxy)-N-(1,1-dimethyl-pent-2-ynyl)-2-methylsulfanyl-acetamide (Compound No3 of Table 2 and No4 of Table 4);
2-(3-Bromo-8-methyl-quinolin-6-yloxy)-N-(5-methoxy-1,1-dimethyl-pent-2-ynyl)-2-methylsulfanyl-acetamide (Compound No24 of Table 2 and No5 of Table 4);
2-(3-Bromo-8-methyl-quinolin-6-yloxy)-N-(6-chloro-1,1-dimethyl-hex-2-ynyl)-2-methylsulfanyl-acetamide (Compound No6 of Table 4);
2-(3-Bromo-8-methyl-quinolin-6-yloxy)-N-(1-ethyl-1-methyl-prop-2-ynyl)-2-methylsulfanyl-acetamide (Compound No7 of Table 4);
2-(3-Bromo-8-methyl-quinolin-6-yloxy)-N-(1-ethyl-1-methyl-but-2-ynyl)-2-methylsulfanyl-acetamide (Compound No8 of Table 4);
N-(1,1-Dimethyl-but-2-ynyl)-2-(3-iodo-8-methyl-quinolin-6-yloxy)-2-methylsulfanyl-acetamide (Compound No2 of Table 3 and No9 of Table 4);
N-(1,1-Dimethyl-prop-2-ynyl)-2-(3-iodo-8-methyl-quinolin-6-yloxy)-2-methylsulfanyl-acetamide (Compound No1 of Table 3 and No10 of Table 4);
2-(3-Iodo-8-methyl-quinolin-6-yloxy)-N-(4-methoxy-1,1-dimethyl-but-2-ynyl)-2-methylsulfanyl-acetamide (Compound No23 of Table 3 and No11 of Table 4);
2-(3-Bromo-8-methyl-quinolin-6-yloxy)-N-(1-isopropyl-1-methyl-prop-2-ynyl)-2-methylsulfanyl-acetamide (Compound No12 of Table 4);
N-(1-Ethyl-1-methyl-prop-2-ynyl)-2-(3-iodo-8-methyl-quinolin-6-yloxy)-2-methylsulfanyl-acetamide (Compound No13 of Table 4);
N-(1-Ethyl-1-methyl-but-2-ynyl)-2-(3-iodo-8-methyl-quinolin-6-yloxy)-2-methylsulfanyl-acetamide (Compound No14 of Table 4);
N-(1,1-Dimethyl-pent-2-ynyl)-2-(3-iodo-8-methyl-quinolin-6-yloxy)-2-methylsulfanyl-acetamide (Compound No3 of Table 3 and No15 of Table 4);
2-(3-Iodo-8-methyl-quinolin-6-yloxy)-N-(5-methoxy-1,1-dimethyl-pent-2-ynyl)-2-methylsulfanyl-acetamide (Compound No24 of Table 3 and No16 of Table 4);
2-(3-Chloro-8-methyl-quinolin-6-yloxy)-N-(1,1-dimethyl-prop-2-ynyl)-2-methylsulfanyl-acetamide (Compound No1 of Table 1 and No17 of Table 4);
2-(3-Chloro-8-methyl-quinolin-6-yloxy)-N-(1,1-dimethyl-but-2-ynyl)-2-methylsulfanyl-acetamide (Compound No2 of Table 1 and No18 of Table 4);
2-(3-Chloro-8-methyl-quinolin-6-yloxy)-N-(4-methoxy-1,1-dimethyl-but-2-ynyl)-2-methylsulfanyl-acetamide (Compound No23 of Table 1 and No19 of Table 4);
2-(3-Chloro-8-methyl-quinolin-6-yloxy)-N-(1,1-dimethyl-pent-2-ynyl)-2-methylsulfanyl-acetamide (Compound No3 of Table 1 and No20 of Table 4);
2-(3-Chloro-8-methyl-quinolin-6-yloxy)-N-(5-methoxy-1,1-dimethyl-pent-2-ynyl)-2-methylsulfanyl-acetamide (Compound No24 of Table 1 and No21 of Table 4);
2-(3-Bromo-8-methyl-quinolin-6-yloxy)-N-(4-ethoxy-1,1-dimethyl-but-2-ynyl)-2-methylsulfanyl-acetamide (Compound No25 of Table 2 and No22 of Table 4);
2-(3-Bromo-8-methyl-quinolin-6-yloxy)-N-(6-chloro-5-hydroxy-1,1-dimethyl-hex-2-ynyl)-2-methylsulfanyl-acetamide (Compound No23 of Table 4) 2-(3-Bromo-8-methyl-quinolin-6-yloxy)-N-((E)-6-hydroxy-1,1-dimethyl-hex-4-en-2-ynyl)-2-methylsulfanyl-acetamide (Compound No24 of Table 4);
2-(3-Bromo-8-methyl-quinolin-6-yloxy)-N-(5-(2-methoxy-ethoxy)-1,1-dimethyl-pent-2-ynyl]-2-methylsulfanyl-acetamide (Compound No43 of Table 2 and No25 of Table 4);
2-(3-Bromo-8-methyl-quinolin-6-yloxy)-N-[1,1-dimethyl-3-(2-methyl-oxiranyl)-prop-2-ynyl]-2-methylsulfanyl-acetamide (Compound No26 of Table 4);
2-(3-Bromo-8-methyl-quinolin-6-yloxy)-N-(5-chloro-4-hydroxy-1,1,4-trimethyl-pent-2-ynyl)-2-methylsulfanyl-acetamide (Compound No27 of Table 4);
N-(4-Allyloxy-1,1-dimethyl-but-2-ynyl)-2-(3-bromo-8-methyl-quinolin-6-yloxy)-2-methylsulfanyl-acetamide (Compound No36 of Table 2 and No28 of Table 4);
2-(3-Bromo-8-methyl-quinolin-6-yloxy)-N-(1,1-dimethyl-4-prop-2-ynyloxy-but-2-ynyl)-2-methylsulfanyl-acetamide (Compound No39 of Table 2 and No29 of Table 4);
2-(3-Bromo-8-methyl-quinolin-6-yloxy)-N-(4-hydroxy-1,1-dimethyl-but-2-ynyl)-2-methylsulfanyl-acetamide (Compound No21 of Table 2 and No30 of Table 4);
2-(3-Bromo-8-methyl-quinolin-6-yloxy)-N-(4-methoxymethoxy-1,1-dimethyl-but-2-ynyl)-2-methylsulfanyl-acetamide (Compound No46 of Table 2 and No31 of Table 4);
2-(3-Bromo-8-methyl-quinolin-6-yloxy)-N-[4-(2-methoxy-ethoxymethoxy)-1,1-dimethyl-but-2-ynyl]-2-methylsulfanyl-acetamide (Compound No55 of Table 2 and No32 of Table 4);

2-(3-Bromo-8-ethyl-quinolin-6-yloxy)-N-(1,1-dimethyl-prop-2-ynyl)-2-methylsulfanyl-acetamide (Compound No33 of Table 4);

2-(3-Bromo-8-ethyl-quinolin-6-yloxy)-N-(1,1-dimethyl-but-2-ynyl)-2-methylsulfanyl-acetamide (Compound No34 of Table 4);

2-(3-Bromo-8-ethyl-quinolin-6-yloxy)-N-(4-methoxy-1,1-dimethyl-but-2-ynyl)-2-methylsulfanyl-acetamide (Compound No35 of Table 4).

EXAMPLE 3

This Example illustrates the fungicidal properties of compounds of formula (1).

The compounds are tested in a leaf disk assay, with methods described below. The test compounds are dissolved in DMSO and diluted into water to 200 ppm. In the case of the test on *Pythium ultimum,* they are dissolved in DMSO and diluted into water to 20 ppm. *Erysiphe graminis f.sp. hordei* (barley powdery mildew): Barley leaf segments are placed on agar in a 24-well plate and sprayed with a solution of the test compound. After allowing to dry completely, for between 12 and 24 hours, the leaf disks are inoculated with a spore suspension of the fungus. After appropriate incubation the activity of a compound is assessed four days after inoculation as preventive fungicidal activity.

*Erysiphe graminis f.sp. tritici* (wheat powdery mildew): Wheat leaf segments are placed on agar in a 24-well plate and sprayed with a solution of the test compound. After allowing to dry completely, for between 12 and 24 hours, the leaf disks are inoculated with a spore suspension of the fungus. After appropriate incubation the activity of a compound is assessed four days after inoculation as preventive fungicidal activity. *Puccinia recondita f.sp. tritici* (wheat brown rust): Wheat leaf segments are placed on agar in a 24-well plate and sprayed with a solution of the test compound. After allowing to dry completely, for between 12 and 24 hours, the leaf disks are inoculated with a spore suspension of the fungus. After appropriate incubation the activity of a compound is assessed nine days after inoculation as preventive fungicidal activity.

*Septoria nodorum* (wheat glume blotch): Wheat leaf segments are placed on agar in a 24-well plate and sprayed with a solution of the test compound. After allowing to dry completely, for between 12 and 24 hours, the leaf disks are inoculated with a spore suspension of the fungus. After appropriate incubation the activity of a compound is assessed four days after inoculation as preventive fungicidal activity.

*Pyrenophora teres* (barley net blotch): Barley leaf segments are placed on agar in a 24-well plate and sprayed with a solution of the test compound. After allowing to dry completely, for between 12 and 24 hours, the leaf disks are inoculated with a spore suspension of the fungus. After appropriate incubation the activity of a compound is assessed four days after inoculation as preventive fungicidal activity.

*Pyricularia oryzae* (rice blast): Rice leaf segments are placed on agar in a 24-well plate and sprayed with a solution of the test compound. After allowing to dry completely, for between 12 and 24 hours, the leaf disks are inoculated with a spore suspension of the fungus. After appropriate incubation the activity of a compound is assessed four days after inoculation as preventive fungicidal activity.

*Botrytis cinerea* (grey mould): Bean leaf disks are placed on agar in a 24-well plate and sprayed with a solution of the test compound. After allowing to dry completely, for between 12 and 24 hours, the leaf disks are inoculated with a spore suspension of the fungus. After appropriate incubation the activity of a compound is assessed four days after inoculation as preventive fungicidal activity.

*Phytophthora infestans* (late blight of potato on tomato): Tomato leaf disks are placed on water agar in a 24-well plate and sprayed with a solution of the test compound. After allowing to dry completely, for between 12 and 24 hours, the leaf disks are inoculated with a spore suspension of the fungus. After appropriate incubation the activity of a compound is assessed four days after inoculation as preventive fungicidal activity. *Plasmopara viticola* (downy mildew of grapevine): Grapevine leaf disks are placed on agar in a 24-well plate and sprayed a solution of the test compound. After allowing to dry completely, for between 12 and 24 hours, the leaf disks are inoculated with a spore suspension of the fungus. After appropriate incubation the activity of a compound is assessed seven days after inoculation as preventive fungicidal activity.

*Pythium ultimum* (Damping off): Mycelial fragments of the fungus, prepared from a fresh liquid culture, are mixed into potato dextrose broth. A solution of the test compound in dimethyl sulphoxide is diluted with water to 20ppm then placed into a 96-well microtiter plate and the nutrient broth containing the fungal spores is added. The test plate is incubated at 24°C and the inhibition of growth is determined photometrically after 48 hours.

The following compounds (number of compound first, followed by table number in brackets) gave at least 60% control of the following fungal infection at 200ppm: *Plasmopara viticola,* compounds 1(4), 2(4), 3(4), 4(4), 5(4), 7(4), 8 (4), 12(4), 17(4), 18(4), 19(4), 21(4), 22(4), 24(4), 25(4), 26(4), 27(4), 32(4), 33(4), 34(4), 35(4), 36(4) *Phytophthora infestans,* compounds 1(4), 2(4), 3(4), 5(4), 17(4), 18(4), 19(4), 20(4), 21 (4), 24(4), 25(4), 26(4), 28(4), 30(4), 31 (4), 32(4), 35(4), 36(4)

*Erysiphe graminis f.sp. tritici,* compounds 1(4), 2(4), 3(4), 5(4), 7(4), 17(4), 18(4), 19(4), 21 (4), 24(4), 27(4), 28(4), 29 (4), 30(4), 31(4), 32(4), 33(4), 34(4), 35(4), 36(4) *Pyricularia* oryzae, compounds 2(4), 3(4), 7(4), 17(4), 18(4), 29(4), 30 (4), 33(4), 35(4)
*Botrytis cinerea,* compounds 1(4), 28(4), 29(4), 30(4)
*Pyrenophora teres,* compounds 2(4), 17(4), 33(4)
*Puccinia recondita f.sp. tritici,* compounds 2(4), 36(4)
*Septoria nodorum,* compound 1(4), 2(4), 3(4), 7(4), 8(4), 12(4), 17(4), 18(4), 28(4), 30(4), 33(4), 35(4), 36(4)

The following compounds (number of compound first, followed by table number in brackets) gave at least 60% control of the following fungal infection at 20ppm:

*Pythium ultimum,* compounds 1 (4), 2(4), 5(4), 17(4), 18(4), 19(4), 21(4), 24(4), 25(4), 26(4), 28(4), 30(4), 31(4), 33 (4), 34(4), 35(4), 36(4)

Comparison of the fungicidal activity of compound No 17 of the Table 4 according to the invention with the structurally closely related compound No 1 of the Table 23 of WO 2004/108663 A1.

| Compound 17(4) according to invention | Compound 1(23) according to WO 2004/108663 A1 |
|---|---|
| 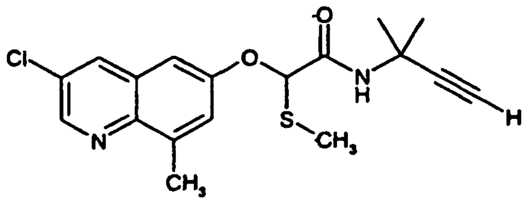 | 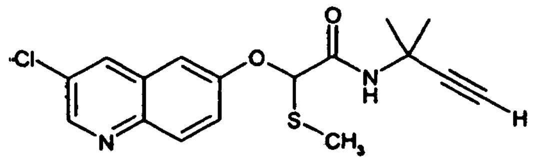 |

Table 5: Activity against *Septoria nodorum*

| Description of test: Wheat leaf segments are placed on agar in a 24-well plate and sprayed with a solution of the test compound. After allowing to dry completely, for between 12 and 24 hours, the leaf disks are inoculated with a spore suspension of the fungus. After appropriate incubation the activity of a compound is assessed four days after inoculation as preventive fungicidal activity. | | |
|---|---|---|
| Concentration (ppm) | Efficacy (%) of compound 17(4) according to invention | Efficacy (%) of compound 1(23) according to WO 2004/108663 |
| 200 | 100 | 0 |
| 60 | 100 | 0 |
| 20 | 80 | 0 |

Table 5 shows that compound No 17 of the Table 4 according to the invention exerts a substantially better fungicidal activity against *Septoria nodorum* than the compound from the state of the art (compound No 1 of the Table 23 described on page 110 of WO 2004/108663 A1). At all application rates, the compound according to the invention is far superior to the compound of the state of the art. This enhanced effect was not to be expected on the basis of the structural similarity of these compounds.

## Claims

**1.** A compound of the general formula (1):

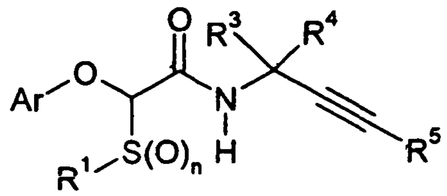

(1) ,

wherein Ar is a group of the formula (A):

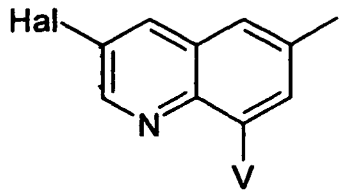

(A) ,

wherein

Hal is chloro, bromo or iodo
V is methyl or ethyl, where V is different from methyl when Hal is iodo,
$R^1$ is methyl or ethyl;
$R^3$ and $R^4$ are independently H, $C_{1-3}$ alkyl, $C_{2-3}$ alkenyl or $C_{2-3}$ alkynyl, or
$R^3$ and $R^4$ join with the carbon atom to which they are attached to form a 4 to 5-membered carbocyclic ring optionally containing one O, S or N atom and optionally substituted with halo or $C_{1-4}$ alkyl;
$R^5$ is H, $C_{1-4}$ alkyl or $C_{3-6}$ cycloalkyl or $C_{3-6}$ cycloalkoxy or $C_{2-4}$ alkenyl, in which the alkyl or cycloalkyl or cycloalkoxy or alkenyl group is optionally substituted with halo, hydroxy, $C_{1-6}$ alkyl, $C_{1-6}$ alkoxy, $C_{1-4}$ alkoxy-$C_{1-4}$ alkoxy, cyano, $C_{3-5}$ alkenyloxy or $C_{3-5}$ alkynyloxy, and n is 0, 1 or 2.

2. A compound according to claim 1. wherein Hal is chloro or bromo.

3. A compound according to claim 1, wherein V is methyl.

4. A compound according to claim 1, wherein $R^3$ and $R^4$ are independently H or $C_{1-3}$ alkyl or $R^3$ and $R^4$ join with the carbon atom to which they are attached to form a 4 or 5-membered carbocyclic ring.

5. A compound according to claim 1, wherein $R^5$ is H, $C_{1-4}$ alkyl, $C_{3-6}$ cycloalkyl, $C_{2-6}$ alkoxyalkyl, $C_{3-6}$ alkenyloxyalkyl, $C_{3-6}$ alkynyloxyalkyl, $C_{1-4}$ hydroxyalkyl. or $C_{1-4}$ haloalkyl.

6. A compound according to claim 1, wherein Hal is bromo, V is methyl, R’ is methyl, n is 0, $R^3$ and $R^4$ are methyl and $R^5$ is H, $C_{1-4}$ alkyl, $C_{3-6}$ cycloalkyl, $C_{1-6}$ alkoxy-$C_{1-4}$ alkyl, $C_{3-6}$ alkenyloxy-$C_{1-4}$ alkyl, $C_{3-6}$ alkynyloxy-$C_{1-4}$ alkyl, $C_{1-4}$ hydroxyalkyl, or $C_{1-4}$ haloalkyl.

7. A process for preparing a compound of the formula (1) according to claim 1, wherein n is 0, which comprises reacting a compound of the formula (7)

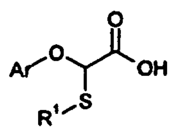

(7) ,

wherein Ar and $R^1$ are as defined in claim 1, with a compound of the formula (8)

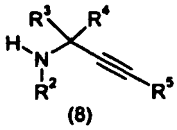

(8) ,

wherein $R^3$, $R^4$ and $R^5$ are as defined in claim 1 and $R^2$ is hydrogen, in the presence of an activating agent.

**8.** A process for preparing a compound of the formula (1) according to claim 1, wherein n is 0, which comprises reacting a compound of the formula (16)

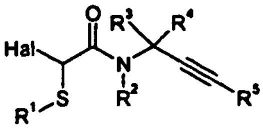

(16) ,

wherein Hal is halogen, $R^1$, $R^3$, $R^4$ and $R^5$ are as defined in claim 1 and $R^2$ is hydrogen, with a compound of the formula ArOH, wherein Ar is as defined in claim 1, in the presence of a base.

**9.** A fungicidal composition comprising a fungicidally effective amount of a compound of formula (1) according to claim 1 and a suitable carrier or diluent therefor.

**10.** A method of combating or controlling phytopathogenic fungi which comprises applying a fungicidally effective amount of a compound of formula (1) according to claim 1 or a composition according to claim 9 to a plant, to a seed of a plant, to the locus of the plant or seed or to soil or any other plant growth medium.

**Patentansprüche**

**1.** Verbindung der allgemeinen Formel (1):

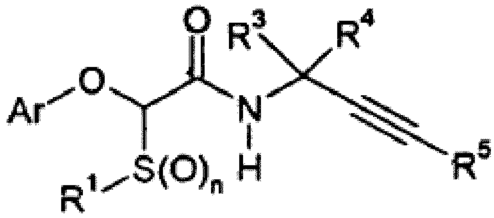

(1) ,

worin A eine Gruppe der Formel (A) bedeutet:

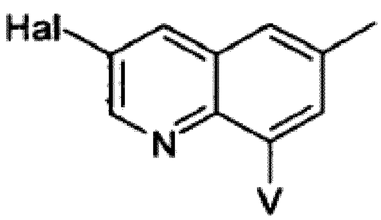

(A) ,

worin

Hal Chlor, Brom oder Iod bedeutet,
V Methyl oder Ethyl bedeutet, wobei, wenn Hal Iod bedeutet, V nicht Methyl bedeutet,
$R^1$ Methyl oder Ethyl bedeutet;
$R^3$ und $R^4$ unabhängig voneinander H, $C_{1-3}$Alkyl, $C_{2-3}$Alkenyl oder $C_{2-3}$Alkinyl bedeuten, oder
$R^3$ und $R^4$ gemeinsam mit dem Kohlenstoffatom, an das sie gebunden sind, einen 4- bis 5-gliedrigen carbocyclischen Ring bilden, der gegebenenfalls ein O-, S- oder N-Atom enthält und gegebenenfalls durch Halogen oder $C_{1-4}$Alkyl substituiert ist;
$R^5$ H, $C_{1-4}$Alkyl oder $C_{3-6}$Cycloalkyl oder $C_{3-6}$Cycloalkoxy oder $C_{2-4}$Alkenyl bedeuten, wobei die Alkyl- oder Cycloalkyl- oder Cycloalkoxy- oder Alkenylgruppe gegebenenfalls durch Halogen, Hydroxy, $C_{1-6}$Alkyl, $C_{1-6}$Alkoxy, $C_{1-4}$Alkoxy-$C_{1-4}$alkoxy, Cyano, $C_{3-5}$Alkenyloxy oder $C_{3-5}$Alkinyloxy substituiert ist, bedeutet,
und n 0, 1 oder 2 bedeutet.

**2.** Verbindung nach Anspruch 1, worin Hal Chlor oder Brom bedeutet.

**3.** Verbindung nach Anspruch 1, worin V Methyl bedeutet.

**4.** Verbindung nach Anspruch 1, worin $R^3$ und $R^4$ unabhängig H oder $C_{1-3}$Alkyl bedeuten oder $R^3$ und $R^4$ gemeinsam mit dem Kohlenstoffatom, an das sie gebunden sind, einen 4- oder 5-gliedrigen carbocyclischen Ring bilden.

**5.** Verbindung nach Anspruch 1, wobei $R^5$ H, $C_{1-4}$Alkyl, $C_{3-6}$Cycloalkyl, $C_{2-6}$Alkoxyalkyl, $C_{3-6}$Alkenyloxyalkyl, $C_{3-6}$Alkinyloxyalkyl, $C_{1-4}$Hydroxyalkyl oder $C_{1-4}$Halogenalkyl bedeutet.

**6.** Verbindung nach Anspruch 1, worin Hal Brom bedeutet, V Methyl bedeutet, $R^1$ Methyl bedeutet, n 0 bedeutet, $R^3$ und $R^4$ Methyl bedeuten und $R^5$ H, $C_{1-4}$Alkyl, $C_{3-6}$Cycloalkyl, $C_{1-6}$Alkoxy-$C_{1-4}$alkyl, $C_{3-6}$Alkenyloxy-$C_{1-4}$alkyl, $C_{3-6}$Alkinyloxy-$C_{1-4}$alkyl, $C_{1-4}$Hydroxyalkyl oder $C_{1-4}$Halogenalkyl bedeutet.

**7.** Verfahren zur Herstellung einer Verbindung der Formel (1) nach Anspruch 1, worin n 0 bedeutet, bei dem man eine Verbindung der Formel (7)

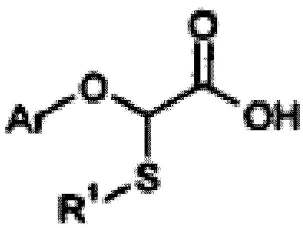

(7) ,

worin Ar und $R^1$ wie in Anspruch 1 definiert sind, in Gegenwart eines Aktivierungsmittels mit einer Verbindung der Formel (8)

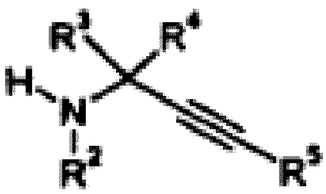

(8) ,

worin $R^3$, $R^4$ und $R^5$ wie in Anspruch 1 definiert sind und $R^2$ Wasserstoff bedeutet, umsetzt.

8. Verfahren zur Herstellung einer Verbindung der Formel (1) nach Anspruch 1, worin n 0 bedeutet, bei dem man eine Verbindung der Formel (16)

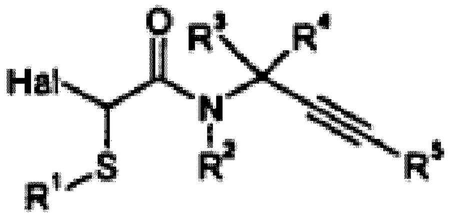

(16)

worin Hal Halogen bedeutet, $R^1$, $R^3$, $R^4$ und $R^5$ wie in Anspruch 1 definiert sind und $R^2$ Wasserstoff bedeutet, in Gegenwart einer Base mit einer Verbindung der Formel ArOH, worin Ar wie in Anspruch 1 definiert ist, umsetzt.

9. Fungizide Zusammensetzung, die eine fungizidwirksame Menge einer Verbindung der Formel (1) nach Anspruch 1 und einen hierfür geeigneten Träger bzw. ein hierfür geeignetes Verdünnungsmittel umfasst.

10. Verfahren zum Bekämpfen oder Kontrollieren von phytopathogenen Pilzen, bei dem man eine fungizidwirksame Menge einer Verbindung der Formel (1) nach Anspruch 1 oder einer Zusammensetzung nach Anspruch 9 auf eine Pflanze, auf einen Samen einer Pflanze, auf den Ort der Pflanze oder des Samens oder auf Boden oder ein beliebiges sonstiges Pflanzenwachstumssubstrat aufbringt.

**Revendications**

1. Composé de formule générale (1) :

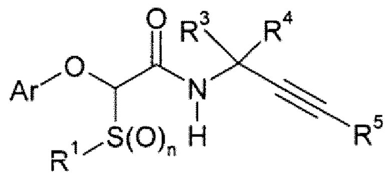

(1) ,

dans laquelle
Ar est un groupement de formule (A) :

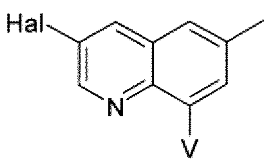

(A) ,

dans laquelle

Hal est chloro, bromo ou iodo ;
V est méthyle ou éthyle, où V est différent de méthyle lorsque Hal est iodo ;
$R^1$ est méthyle ou éthyle ;
$R^3$ et $R^4$ sont indépendamment H, $C_{1-3}$ alkyle, $C_{2-3}$ alcényle ou $C_{2-3}$ alcynyle, ou $R^3$ et $R^4$ se lient avec l'atome de carbone auquel ils sont fixés pour former un cycle carbocyclique de 4 à 5 chaînons contenant éventuellement

un atome de 0, S ou N et éventuellement substitué par halogéno ou $C_{1-4}$ alkyle ;
$R^5$ est H, $C_{1-4}$ alkyle ou $C_{3-6}$ cycloalkyle ou $C_{3-6}$ cycloalcoxy ou $C_{2-4}$ alcényle, où le groupement alkyle ou cycloalkyle ou cycloalcoxy ou alcényle est éventuellement substitué par halogéno, hydroxy, $C_{1-6}$ alkyle, $C_{1-6}$ alcoxy, $C_{1-4}$ alcoxy-$C_{1-4}$ alcoxy, cyano, $C_{3-5}$ alcényloxy ou $C_{3-5}$ alcynyloxy, et
n vaut 0, 1 ou 2.

**2.** Composé selon la revendication 1, dans lequel Hal est chloro ou bromo.

**3.** Composé selon la revendication 1, dans lequel V est méthyle.

**4.** Composé selon la revendication 1, dans lequel $R^3$ et $R^4$ sont indépendamment H ou $C_{1-3}$ alkyle ou $R^3$ et $R^4$ se lient avec l'atome de carbone auquel ils sont fixés pour former un cycle carbocyclique de 4 ou 5 chaînons.

**5.** Composé selon la revendication 1, dans lequel $R^5$ est H, $C_{1-4}$ alkyle, $C_{3-6}$ cycloalkyle, $C_{2-6}$ alcoxyalkyle, $C_{3-6}$ alcényloxyalkyle, $C_{3-6}$ alcynyloxyalkyle, $C_{1-4}$ hydroxyalkyle, ou $C_{1-4}$ halogénoalkyle.

**6.** Composé selon la revendication 1, dans lequel Hal est bromo, V est méthyle, $R^1$ est méthyle, n vaut 0, $R^3$ et $R^4$ sont méthyle et $R^5$ est H, $C_{1-4}$ alkyle, $C_{3-6}$ cycloalkyle, $C_{1-6}$ alcoxy-$C_{1-4}$ alkyle, $C_{3-6}$ alcényloxy-$C_{1-4}$ alkyle, $C_{3-6}$ alcynyloxy-$C_{1-4}$ alkyle, $C_{1-4}$ hydroxyalkyle, ou $C_{1-4}$ halogénoalkyle.

**7.** Procédé de préparation d'un composé de formule (1) selon la revendication 1, dans laquelle n vaut 0, comprenant la réaction d'un composé de formule (7)

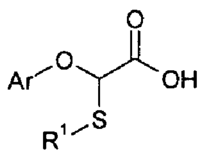

(7) ,

dans laquelle Ar et $R^1$ sont tels que définis selon la revendication 1, avec un composé de formule (8)

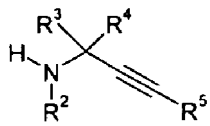

(8) ,

dans laquelle $R^3$, $R^4$ et $R^5$ sont tels que définis selon la revendication 1 et $R^2$ est hydrogène, en présence d'un agent activant.

**8.** Procédé de préparation d'un composé de formule (1) selon la revendication 1, dans laquelle n vaut 0, comprenant la réaction d'un composé de formule (16)

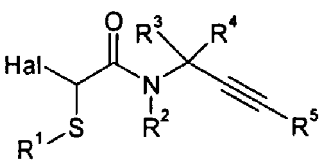

(16) ,

dans laquelle Hal est halogène, $R^1$, $R^3$, $R^4$ et $R^5$ sont tels que définis selon la revendication 1 et $R^2$ est hydrogène, avec un composé de formule ArOH, où Ar est tel que défini selon la revendication 1, en présence d'une base.

**9.** Composition fongicide comprenant une quantité efficace sur le plan fongicide d'un composé de formule (1) selon la revendication 1 et un véhicule ou diluant convenable pour celui-ci.

**10.** Méthode pour lutter contre ou contrôler des champignons phytopathogènes, comprenant l'application d'une quantité efficace sur le plan fongicide d'un composé de formule (1) selon la revendication 1, ou d'une composition selon la revendication 9, à une plante, aux semences d'une plante, au lieu où se développent la plante ou les semences, ou au sol ou à tout autre milieu de croissance de la plante.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 04108663 A
- EP 0834498 A
- WO 2006058700 A1
- WO 2004108663 A1
- WO 2004108663 A